(19) Europäisches Patentamt
European Patent Office
Office européen des brevets

(11) **EP 4 249 751 B1**

(12) **EUROPÄISCHE PATENTSCHRIFT**

(45) Veröffentlichungstag und Bekanntmachung des Hinweises auf die Patenterteilung:
**07.05.2025 Patentblatt 2025/19**

(21) Anmeldenummer: **23160540.3**

(22) Anmeldetag: **07.03.2023**

(51) Internationale Patentklassifikation (IPC):
**F04B 43/12** (2006.01)     **F04B 49/08** (2006.01)

(52) Gemeinsame Patentklassifikation (CPC):
**F04B 43/12; F04B 49/08**

(54) **REGELEINRICHTUNG FÜR EINE PERISTALTIKPUMPE, PERISTALTIKPUMPE, INJEKTIONSGERÄT UND VERFAHREN ZUR STEUERUNG EINER PERISTALTIKPUMPE**

CONTROL DEVICE FOR A PERISTALTIC PUMP, PERISTALTIC PUMP, INJECTION DEVICE AND METHOD FOR CONTROLLING A PERISTALTIC PUMP

DISPOSITIF DE RÉGULATION POUR POMPE PÉRISTALTIQUE, POMPE PÉRISTALTIQUE, APPAREIL D'INJECTION ET PROCÉDÉ DE COMMANDE DE POMPE PÉRISTALTIQUE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **25.03.2022 DE 102022107119**

(43) Veröffentlichungstag der Anmeldung:
**27.09.2023 Patentblatt 2023/39**

(73) Patentinhaber: **ulrich GmbH & Co. KG 89081 Ulm (DE)**

(72) Erfinder:
• **Becker, Andreas 89075 Ulm (DE)**
• **Seibold, Felix 89081 Ulm (DE)**

(74) Vertreter: **Charrier Rapp & Liebau Patentanwälte PartG mbB Fuggerstraße 20 86150 Augsburg (DE)**

(56) Entgegenhaltungen:
CN-B- 104 739 520     SE-C2- 537 628
US-B2- 9 567 992

EP 4 249 751 B1

**Beschreibung**

Die Erfindung betrifft eine Regeleinrichtung für eine Peristaltikpumpe nach dem Oberbegriff des Anspruchs 1, eine Peristaltikpumpe mit einer solchen Regeleinrichtung, ein Injektionsgerät mit einer solchen Peristaltikpumpe und ein Verfahren zur Steuerung einer Peristaltikpumpe.

Injektionsgeräte sind medizinische Geräte, mittels derer flüssige Injektionsmittel (Medium) über eine Pumpvorrichtung kontrolliert in einen menschlichen oder tierischen Körper eingebracht werden kann. Bei dem Injektionsmittel kann es sich bspw. um ein Kontrastmittel zur Erhöhung des Kontrasts in einem bildgebenden Verfahren, wie der Computertomographie oder der Magnetresonanztomographie, handeln. Neben einer Kontrolle des Injektionsvolumens sowie einer Durchflussrate (Volumenstrom) des Injektionsmittels ist es notwendig, den Druck in einer Ausgangsleitung zu überwachen, da zu hohe Drücke einerseits schädlich für den Körper sein und/oder das Injektionsgerät schädigen können. Aus diesem Grund sehen bekannte Injektionsgeräte Steuerungen oder Regelungen vor, die das Injektionsgerät bzw. dessen Pumpvorrichtung bei Überschreitung einer noch zulässigen Druckgrenze (maximaler Soll-Druck), der unterhalb eines definierten Gefährdungsdrucks liegt, abschalten. Gerade bei zyklisch oszillierenden Druckverläufen, die z.B. bei Axialkolben- oder Rollenpumpen vorliegen, können Druckmaxima auftreten, die nur kurzzeitig und geringfügig über dem Druckgrenzwert liegen und deshalb zu einem unnötigen Abschalten der Pumpvorrichtung bzw. einem Abbruch des Injektionsvorgangs führen. Dies verlängert den Injektionsvorgang und erhöht unnötig das insgesamt injizierte Volumen an Injektionsmittels.

Die US 9 567 992 B2 offenbart eine Vorrichtung bestehend aus einer Pumpe zur Förderung einer Flüssigkeit und einer die Pumpe steuernde Steuereinheit sowie eine Medikamentendosiervorrichtung, die aufgrund von Druckpulsen in der Flüssigkeit eine Medikamentendosis abgibt, wobei die Steuereinheit dazu eingerichtet ist, die Abgabe der Medikamentendosis durch Änderung des Arbeitspunktes der Pumpe kontrolliert zu verursachen, wobei die Pumpe eine peristaltische Pumpe mit einem Rotor sein kann und die Steuereinheit dazu eingerichtet ist, den Arbeitspunkt, der peristaltischen Pumpe in Abhängigkeit des Winkels, den der Rotor mit einem beliebigen feststehenden Punkt einschließt, zu ändern. Als Arbeitspunkt, wird dabei zumindest ein Betriebsparameter der peristaltischen Pumpe, wie z.B. Eingangs- und Ausgangsdruck in der gepumpten Flüssigkeit, Förderraten der gepumpten Flüssigkeit am Eingang und am Ausgang der Pumpe, Winkelgeschwindigkeit des Rotors der peristaltischen Pumpe, Winkel des Rotors der peristaltischen Pumpe bezüglich des feststehenden Punkts, sowie Versorgungsspannung, Versorgungsstrom, und Leistungsaufnahme des die Pumpe antreibenden Elektromotors, verstanden. Durch die kontrollierte Änderung des Arbeitspunktes der Pumpe können Druck- oder Flussspitzen im Fördermedium der Pumpe vermieden werden.

Zur Vermeidung von vorschnellen Abbrüchen eines Injektionsvorgangs wird in der US 6 673 033 B1 vorgeschlagen, eine Druckzwischenschwelle zu definieren, bei deren Überschreitung die Leistung einer Pumpvorrichtung zunächst gedrosselt wird, und die Pumpvorrichtung erst abzuschalten, wenn danach trotz gedrosselter Leistung der Druck in eine Schlauchleitung eines Injektors über einen Druckgrenzwert ansteigt.

Ebenso ist es aus der DE 10 2013 113 387 A1 bekannt, bei einer Peristaltikpumpe anstelle eines harten Druckgrenzwerts als Kriterium für den Abbruch eines Injektionsvorgangs ein zeitliches Integral des Druckverlaufs ab Überschreiten eines Druckgrenzwertes als Maßstab für einen Abbruch des Injektionsvorgangs heranzuziehen, so dass kurzzeitige Druckspitzen toleriert werden können und ein nur kurzzeitiges Überschreiten des Druckgrenzwerts nicht zu einem sofortigen Abbruch des Injektionsvorgangs führt.

Nachteilig bei den aus dem Stand der Technik bekannten Regelungsverfahren für Injektionsgeräte ist, dass große Sicherheitsintervalle eingehalten werden müssen - d.h. die Druckgrenze muss deutlich unterhalb des Gefährdungsdrucks liegen - so dass eine ausreichende Reaktionszeit besteht, um die Pumpvorrichtung bei Überschreiten der Druckgrenze herunterregeln bzw. abschalten zu können.

Vor diesem Hintergrund ist es Aufgabe der Erfindung, eine verbesserte Regeleinrichtung für eine Peristaltikpumpe mit einem oszillierenden Druckverlauf (Druckpulsation), eine Peristaltikpumpe mit einer solchen Regeleinrichtung, ein Injektionsgerät mit einer solchen Peristaltikpumpe sowie ein Regelungsverfahren anzugeben, bei denen Sicherheitsintervalle reduziert und eine vorgegebene Druckgrenze näher an einen Gefährdungsdruck herangeführt werden können, ohne ein Schädigungsrisiko für den Patienten und/oder das Material des Injektionsgeräts zu erhöhen. Gleichzeitig sollen Druckpulsationen verringert werden.

Diese Aufgabe wird u.a. gelöst durch die Regeleinrichtung des Anspruchs 1, die Peristaltikpumpe gemäß Anspruch 12, einem Injektionsgerät nach Anspruch 14 sowie einem Regelungsverfahren nach Anspruch 15.

Die erfindungsgemäße Regeleinrichtung dient zur Regelung einer Peristaltikpumpe mit einem Quetschschlauch und sich zyklisch bewegenden Förderelementen zur Förderung eines in dem Quetschschlauch geführten Mediums während eines Fördervorgangs mit einem kontrollierten Volumenstrom in eine mit dem Quetschschlauch verbundene Ausgabeleitung. Dabei komprimieren die Förderelemente den Quetschschlauch zyklisch, so dass sich in der Ausgabeleitung ein Druck mit einem Druckverlauf einstellt, der zyklisch sich wiederholende Druckzyklen aufweist. Jeder Druckzyklus kann so definiert werden, dass er sich bspw. von einem Druckminimum über einen Druckanstieg zu einem Druckmaximum erstreckt und anschließend wieder auf ein Druckminimum eines darauffolgenden Druckzyklus abfällt. Die erfindungsgemäße Regeleinrichtung regelt dabei eine Geschwindigkeit der Peristaltikpumpe - im Falle einer Rollenpumpe kann es sich um eine Winkelgeschwindigkeit eines Förderelements handeln - so, dass ein maximaler Volumenstrom erreicht wird, ohne dass dabei eine Druckgrenze in der Ausgabeleitung überschritten wird. Bei der Druckgrenze kann es sich um einen gewünschten, noch zulässigen Soll-Druck handeln. Die Regeleinrichtung weist hierzu zumindest einen ersten Regelkreis zur Regelung des maximalen Volumenstroms auf. Dieser erhält als extern vorgegebene Führungsgrößen einen Soll-Volumenstrom und die Druckgrenze. Die Regeleinrichtung ist dabei eingerichtet, ein Regelverfahren auszuführen, bei dem für jeden Druckzyklus ein Vorhersagedruck für einen zu erwartenden Maximaldruck innerhalb des Druckzyklus auf Basis mindestens des Drucks in der Ausgabeleitung berechnet wird und der maximale Volumenstrom unter Berücksichtigung des Vorhersagedrucks so begrenzt wird, dass der Druck in der Ausgabeleitung die Druckgrenze in dem Druckzyklus nicht überschreitet.

Zweckmäßig erfasst der erste Regelkreis den aktuellen Druck in der Ausgabeleitung als eine Rückführungsgröße, anhand derer ein Regelteil des Regelkreises den maximalen Volumenstrom vorausschauend regelt.

Durch eine vorausschauende Regelung kann die Geschwindigkeit der Peristaltikpumpe, insb. der Förderelemente der Peristaltikpumpe, bei drohender Überschreitung der Druckgrenze frühzeitig und proaktiv angepasst und insb. reduziert werden. Da durch die Druckvorhersage Reaktionszeiträume verlängert werden, steigen Sicherheitsreserven. Noch zulässige Druckgrenzen können dann näher an kritische Druckwerte, wie z.B. einen Gefährdungsdruck, herangeführt werden, was wiederum höhere Volumenströme und damit z.B. eine Verkürzung einer Injektionsdauer für ein bestimmtes Injektionsvolumen bedeutet. Da bei einer vorausschauenden Regelung die Bedeutung einer reaktionsschnellen Mechanik an Bedeutung verliert, können außerdem verstärkt reaktionsträge Komponenten für die Peristaltikpumpe genutzt werden, wodurch die Herstellung der Peristaltikpumpe billiger wird. Eine Kalibrierung des Regelverfahrens ist bei geeigneter Auslegung der Regeleinrichtung nicht notwendig.

In einer bevorzugten Ausführung der Erfindung umfasst der erste Regelkreis eine Druckphasenerkennung, durch die das Ende eines vorangehenden Druckzyklus und der Beginn eines darauffolgenden Druckzyklus erkannt wird. Die Erkennung kann anhand einer charakteristischen Größe des Druckverlaufs in der Ausgabeleitung, insbesondere einem definierten Druckabfall gegenüber einem Druckmaximum des vorhergehenden Druckzyklus, erfolgen. Der Beginn eines Druckzyklus kann bspw. zu dem Zeitpunkt definiert sein, bei dem ein Druckabfall gegenüber dem (absoluten) Druckmaximum des vorhergehenden Druckzyklus um mehr als einen bestimmten relativen Betrag, bspw. etwa um mindestens 1/6, ¼ oder 1/3 oder - unabhängig von dem vorhergehenden Druckmaximum -um einen absoluten Betrag, z.B. mindestens 2bar, auftritt. Der Druckabfall wird dabei vorteilhaft so groß gewählt, dass dieser Druckabfall nur einmal gegen Ende eines Druckzyklus auftritt. Das Auftreten dieses Druckabfalls ist dann hinreichend robust, um den Beginn eines darauffolgenden Druckzyklus anzuzeigen. Sollte ein typischer Druckzyklus größere Druckschwankungen aufweisen, können ersatzweise größere Druckabfälle und/oder inkrementelle Zähler verwendet werden, die die Anzahl von Druckabfällen um einen bestimmten Mindestbetrag zählen, um so den Beginn eines nachfolgenden Druckzyklus zu erkennen.

Zu Beginn jedes Druckzyklus wird die Regelung des maximalen Volumenstroms neu eingeleitet, und insb. zwischengespeicherte Werte, wie ein zwischengespeichertes Druckmaximum des vorhergehenden Druckzyklus, aktualisiert.

Für eine weitergehende Verbesserung der Regeleinrichtung ist vorteilhaft vorgesehen, dass jeder Druckzyklus anhand vordefinierter Charakteristika in sukzessive aufeinanderfolgende Druckphasen eingeteilt ist und die Druckphasenerkennung den Beginn der einzelnen Druckphasen erkennt, wobei der Vorhersagedruck zur Regelung des maximalen Volumenstroms in dem ersten Regelkreis nur in bestimmten Druckphasen verwendet und in anderen Druckphasen ignoriert bzw. nicht verwendet wird. Die Regelung ist damit unstetig.

Ein Vorteil einer solchen druckphasenabhängigen Regelung liegt darin, dass die Druckvorhersage genau in den Druckphasen eingesetzt werden kann, bei denen die Entwicklung des Drucks in der Ausgabeleitung dynamisch ist und eine Überschreitung der Druckgrenze vergleichsweise plötzlich eintreten kann, so dass in diesen Druckphasen der maximale Volumenstrom konservativ geregelt wird, während er in anderen Druckphasen aggressiver geregelt werden kann, da ein plötzliches Überschreiten der Druckgrenze in diesen anderen Druckphasen nicht zu erwarten ist. Ein weiterer Vorteil ist, dass für unterschiedliche Druckphasen unterschiedliche Regelungen mit z.B. Zuschaltung oder Abschaltung zusätzlicher Regelkreise, die dem ersten Regelkreis überlagert werden und/oder der Veränderung der Regelparameter des ersten Regelkreises, etwa darin verwendeter Verstärkungsfaktoren o.ä., realisiert werden können.

Bei den vorgenannten, vordefinierten Charakteristika kann es sich um absolute oder relative Druckänderungen und/oder um eine absolute oder relative Druckänderungsrate des Drucks in der Ausgabeleitung handeln. Bei den vordefinierten Charakteristika muss es sich nicht zwingend um punktuelle Werte handeln. Es ist bspw. möglich, gleitende Durchschnitte über bestimmte Zeitspannen, wie etwa 0,1, 0,2 oder 0,5 Sekunden, zu wählen.

Die Erkennung der einzelnen Druckphasen durch die Druckphasenerkennung kann anhand der vordefinierten Charakteristika erfolgen. Alternativ können einzelne Druckphasen jedoch auch anhand einer Position der Förderelemente der Peristaltikpumpe durch die Druckphasenerkennung erkannt werden, da die Positionen der Förderelemente - wie weiter unten noch näher erläutert -mit den einzelnen Druckphasen korrelieren. Es ist ebenso möglich, die Erkennung anhand vordefinierter Charakteristika sowie einer Positionsbestimmung der Förderelemente für die Druckphasenerkennung zu kombinieren, sei es zeitgleich, um die Druckphasenerkennung einzelner Druckphasen redundant auszugestalten oder sei es, um einzelne Druckphasen anhand der definierten Charakteristika und andere Druckphasen anhand der Position der Förderelemente zu bestimmen. Bevorzugt ist die Druckphasenerkennung zur Erhöhung der Zuverlässigkeit redundant ausgeführt. Hierdurch können eine Fehleranfälligkeit und insb. Anforderungen an die Ausfallsicherheit einzelner Komponenten, und dadurch Bauteilkosten, reduziert werden.

In einer Ausführungsvariante der Erfindung ist der Druckzyklus durch fünf aufeinanderfolgende Druckphasen gekennzeichnet, wobei eine erste Druckphase durch einen schnellen Druckverlust, eine zweite Druckphase durch einen schnellen Druckanstieg, eine dritte Druckphase durch einen flachen Druckanstieg, eine vierte Druckphase durch einen moderaten Druckanstieg und eine fünfte Druckphase durch ein Druckplateau mit im Wesentlichen konstantem Druck gekennzeichnet ist, und jede Druckphase durch die Druckphasenerkennung erkannt wird. Eine derartige Einteilung eines Druckzyklus ist eine gute Annäherung für Druckzyklen in Injektionsgeräten zur intravenösen Injektion von z.B. Kontrastmitteln. Für die einzelnen Druckphasen sind dabei die statischen und dynamischen Druckwiderstände in der Ausgabeleitung (und den sich anschließenden Leitungen) verantwortlich, die sich insb. durch sich öffnende und schließende Rückschlagventile, Drosseln, den Massenträgheiten des zu fördernden Mediums und anderen Einflüssen ergeben. Für andere Anwendungen können die Druckphasen auch anders als in obigem Beispiel definiert werden.

Bevorzugt ist, wenn im ersten Regelkreis der Vorhersagedruck zur Regelung des maximalen Volumenstroms nur in der vierten Druckphase, d.h. der Druckphase vor dem Druckplateau mit dem (absoluten) Druckmaximum innerhalb des Druckzyklus, verwendet wird. Die vierte Druckphase ist vorteilhaft in einem Bereich definiert, der sich von etwa 50% bis etwa 80% eines Phasenfortschritts des Druckzyklus bewegt. Unter Phasenfortschritts des Druckzyklus bewegt. Unter Phasenfortschritt ist der Druckzyklus in zeitlicher Auflösung zu verstehen, d.h. der Druckzyklus beginnt bei 0% Phasenfortschritt - dies entspricht z.B. einem Druckminimum - und endet bei 100% - dies entspricht dann dem Druckminimum des darauffolgenden Druckzyklus. Da der Vorhersagedruck in der vierten Druckphase zur Berechnung des maximalen Volumenstroms auf den ersten Regelkreis ,aufgeschaltet' wird, und ansonsten 'abgeschaltet' ist, handelt es sich um einen unstetigen Regler. Durch eine derartige Beschränkung der Verwendung des Vorhersagedrucks kann auf eine aufwendige Berechnung des Vorhersagedrucks in den anderen Druckphasen verzichtet werden.

Der Vorhersagedruck kann in einer einfachen und daher vorzugswürdigen Variante über eine lineare Extrapolation des aktuellen Druckanstiegs in der Ausgabeleitung zu einem bestimmten (rechnerischen) Phasenfortschritt, der bevorzugt im Bereich von 80% bis 95% des gesamten Phasenfortschritts liegt und z.B. bei 80%, 90% oder 95% des gesamten Phasenfortschritts und insb. bei 87% des gesamten Phasenfortschritts liegen kann, bestimmt werden. Bei Verwendung des Vorhersagedrucks nur in der vierten Druckphase ist die Vorhersagegenauigkeit einer linearen Extrapolation selbst bei einem stark oszillierenden Druckverlauf eines Druckzyklus hinreichend genau. Auch andere Berechnungsverfahren zur Bestimmung des Vorhersagedrucks, insb. eine Extrapolation auf Basis einer Zeitreihe, sind möglich.

Der bestimmte (rechnerische) Phasenfortschritt, der zur Bestimmung des Vorhersagedrucks verwendet wird, ist dabei zweckmäßig fest gewählt und liegt z.B. bei konstant 87% des gesamten Phasenfortschritts. Dieser bestimmte Phasenfortschritt kann - muss jedoch nicht - mit dem realen Phasenfortschritt übereinstimmen, bei dem reale Druckmaxima in den Druckzyklen auftreten. Tatsächliche Druckmaxima können also bei tatsächlichen Phasenfortschritten auftreten, die vor oder auch hinter dem bestimmten (rechnerischen) Phasenfortschritt von im Beispiel 87% des gesamten Phasenfortschritts liegen.

Der bestimmte Phasenfortschritt ist bevorzugt rechnerisch so gewählt und eingestellt, dass Vorhersagedrücke erzielt werden, die möglichst genau die tatsächlichen Druckmaxima von aufeinanderfolgenden Druckzyklen wiedergeben. Der bestimmte (rechnerische) Phasenfortschritt, zu dem der Vorhersagedruck extrapoliert wird, kann dabei verschoben zu dem realen Phasenfortschritt sein, bei dem ein reales Druckmaximum auftritt, wenn sich hierdurch bessere Vorhersagedrücke ergeben.

In einer konkretisierten Anwendung der Erfindung regelt die Regeleinrichtung eine Rollenpumpe. Rollenpumpen haben den Vorteil nicht auftretender Richtungswechsel, weshalb sich Rollenpumpen besonders gut für eine genaue Dosierung von Injektionsboli in Injektionsgeräten eignen. Da das geförderte Medium bei Rollenpumpen in einem Quetschschlauch gefördert wird, findet in Rollenpumpen außerdem kein unmittelbarer Kontakt zwischen dem geförderten Medium und der Pumpe statt, was aus hygienischen Gründen vorteilhaft ist. Weiterhin kann der Quetschschlauch einfach ausgetauscht werden.

Gegenstand der Erfindung ist daher auch eine Rollenpumpe mit einem drehbaren Rotor, einer Mehrzahl von Förderelementen zum Quetschen eines Quetschschlauchs, die als an dem Rotor angeordnete Rollen (Quetschrollen) ausgebildet sind, wobei der Quetschschlauch entlang eines Schlauchbetts mit einem Einlaufbereich und einem Auslaufbereich gelagert ist und die Förderelemente bei Drehung des Rotors nacheinander jeweils einen Abschnitt des Quetschschlauchs beim Eintreten der Förderelemente in das Schlauchbett auf Höhe des Einlaufbereichs und bis zum Austreten der Förderelemente aus dem Schlauchbett auf Höhe des Auslaufbereichs fluiddicht quetschen und dadurch ein in dem Quetschschlauch befindliches Medium in Drehrichtung entgegen einem Staudruck in einer mit dem Quetschschlauch verbundenen Ausgabeleitung in die Ausgabeleitung fördern, wobei die Rollenpumpe eine erfindungsgemäße Regeleinrichtung enthält.

Dabei wird jeweils ein vor dem Auslaufbereich befindliches Volumen des Quetschschlauchs von einem maximalen Volumen auf ein minimales Volumen komprimiert, solange bis ein Förderelement auf Höhe des Auslaufbereichs aus dem Schlauchbett austritt, den durch dieses Förderelement gequetschten Abschnitt des Quetschlauchs freigibt und damit das vor dem Auslaufbereich befindliche Volumen des Quetschschlauchs von dem minimalen Volumen auf das maximale Volumen vergrößert. Die Volumenreduktion und Volumenvergrößerung des vor dem Auslaufbereich befindlichen Volumens stellen gemeinsam einen vollständigen Druckzyklus dar.

Die Rollenpumpe weist ferner n vorzugsweise radial gleichverteilte Förderelemente auf, so dass die Rollenpumpe bei einer vollständigen Drehung des Rotors um 360° n Pumpenhübe mit n Druckzyklen ausführt. Die Anzahl der Rollenelemente beträgt z.B. drei.

Da jedes Förderelement nur in einem bestimmten Winkelabschnitt, nämlich in dem Winkelabschnitt in dem das Förderelement die "führende" Rolle in Bezug auf den Auslaufbereich bzw. die Ausgabeleitung darstellt, an einem Druckzyklus beteiligt ist, korreliert in diesem Winkelabschnitt die Winkelposition des Förderelements mit dem Phasenfortschritt des Druckzyklus. Dadurch korrelieren auch einzelne Winkelbereiche der Winkelposition der Förderelemente innerhalb des Winkelabschnitts mit einzelnen Druckphasen eines Druckzyklus.

Dies ermöglicht vorteilhaft eine Bestimmung der Druckphase anhand der Winkelposition eines der Förderelemente, insb. eines 'führenden' Förderelements. Anstelle einer fehleranfälligen Druckphasenerkennung über vordefinierte Charakteristika, die unzuverlässig sein können, ist es zweckmäßig, wenn die Druckphasenerkennung der Regeleinrichtung den Beginn mindestens einer Druckphase eines Druckzyklus über eine Winkelposition eines Förderelements bestimmt.

Um auf Positionssensoren zur Ermittlung der Winkelposition der Förderelemente verzichten zu können, wird die Winkelposition zweckmäßig indirekt z.B. über die zeitliche Integration der Winkelgeschwindigkeit der Förderelemente bzw. des Rotors der Rollenpumpe ermittelt. Die Winkelgeschwindigkeit des Rotors steht üblicherweise unmittelbar über ein Steuergerät der Rollenpumpe zur Verfügung. Wie weiter oben dargestellt, kann über einen Druckabfall um einen Mindestbetrag eine absolute Winkelposition des führenden Förderelements angenähert werden.

In einer weiterführenden Ausgestaltung der Erfindung umfasst der erste Regelkreis einen Mittelwert-Filter, das den maximalen Volumenstrom in Abhängigkeit eines in der Regeleinrichtung zwischengespeicherten Maximaldrucks eines dem aktuellen Druckzyklus unmittelbar vorhergehenden Druckzyklus anpasst. Hierdurch können Fehler in der Messung des Drucks in der Ausgabeleitung ausgeglichen werden. Außerdem kann durch den Mittelwert-Filter das Schwingungsverhalten des Regelkreises gedämpft und insb. ein Überschwingen der Regelgröße verhindert oder zumindest reduziert werden. Zweckmäßig wird hierzu der vorhergehende, gemessene Maximaldruck als Verhältnis von dem gemessenen Maximaldruck zu der Druckgrenze (Soll-Druck) in den ersten Regelkreis zurückgeführt und hierauf basierend ein neuer Soll-Volumenstrom eingestellt. Eine Abweichung zwischen dem tatsächlich auftretenden Maximaldruck und der Druckgrenze (Soll-Druck) kann hierdurch mit jedem Zyklus verringert werden.

Vorteilhaft ist der erste Regelkreis ein PID-Regler mit einem Proportional-Glied, einem Integral-Glied und einem Differential-Glied. Durch einen PID-Regler ist eine schnelle und genaue Annäherung der Regelgröße an die Führungsgrößen möglich.

In dem Differential-Glied wird vorzugsweise der Vorhersagedruck zur Regelung des maximalen Volumenstroms verarbeitet. In dem Differential-Glied können jedoch auch weitere Rückführungsgrößen, z.B. eine Änderungsrate einer Drehgeschwindigkeit eines Rotors einer Peristaltikpumpe und/oder eine Änderungsrate des Volumenstroms berücksichtigt werden.

Das Differential-Glied wird vorteilhaft in bestimmten Druckphasen zu 0 gesetzt. Hierdurch kann ein vorauseilender Regeleingriff bedarfsweise ausgeschaltet werden.

Zusätzlich kann die Regeleinrichtung eine Steuerung zur Glättung von Druckspitzen des Drucks in Druckzyklen umfassen. Die Steuerung ist vorteilhaft ein Geschwindigkeitsadapter.

Der Geschwindigkeitsadapter ist bevorzugt so eingerichtet, dass jeweils zu einem bestimmten Fortschritt des Druckzyklus eine Soll-Geschwindigkeit eines Förderelements der Peristaltikpumpe gegenüber einer durchschnittlichen Soll-Geschwindigkeit vor Abschluss eines Druckzyklus um einen bestimmten Betrag auf eine erniedrigte Soll-Geschwindigkeit abgesenkt und/oder nach Abschluss eines Druckzyklus um einen bestimmten Betrag auf eine erhöhte Soll-Geschwindigkeit erhöht und für jeweils eine bestimmte Haltedauer gehalten wird.

Da bei einem Übergang von einem Druckzyklus zum nächsten Druckzyklus der Druck - und damit der Förderaufwand - rasch abfällt, ändert sich auch der Leistungsbedarf der Peristaltikpumpe schlagartig. Um einen konstanten Soll-Volumenstrom zu halten, müsste die Leistung der Peristaltikpumpe beim Übergang zwischen einem vorhergehenden und dem nachfolgenden Druckzyklus daher reduziert oder sogar umgekehrt (gebremst) werden. Dies ist nicht nur energetisch nachteilig. Mit der vorgeschlagenen Steuerung ist eine kurzzeitige Erhöhung der Drehgeschwindigkeit der Peristaltikpumpe bzw. der Förderelemente respektive ein kurzzeitig erhöhter maximaler Volumenstrom beim Wechsel des Druckzyklus zulässig. Durch die Erhöhung des Soll-Volumenstroms bzw. der Drehgeschwindigkeit zum Beginn eines Druckzyklus kann der Druckabfall reduziert und im Anschluss ein für die Injektion notwendiges Druckniveau schneller erreicht werden. Durch die Reduzierung des Soll-Volumenstroms bzw. der Drehgeschwindigkeit zum Ende eines Druckzyklus kann der Druckanstieg zeitlich gedehnt werden. Dies schafft zusätzliche Sicherheitsreserven.

Die Geschwindigkeit der Förderelemente erhöht sich nach Abschluss eines Druckzyklus sprungartig auf die erhöhte Soll-Geschwindigkeit und wird nach der Haltedauer anschließend linear bis auf die erniedrigte Soll-Geschwindigkeit des Förderelements abgesenkt. Hierdurch können Lastspitzen reduziert werden.

Alle Haltedauern können gleich oder unterschiedlich lang sein. Die Erhöhung und Erniedrigung der Soll-Geschwindigkeit ist zweckmäßigerweise betragsmäßig gleich groß. Die Geschwindigkeit der Peristaltikpumpe kann beispielsweise prozentual um einen vorgegebenen Anteil der mittleren Soll-Geschwindigkeit erhöht oder erniedrigt werden, wobei die Änderung der Geschwindigkeit bevorzugt zwischen 20% und 40% der mittleren Soll-Geschwindigkeit liegt und insbesondere bei 20%, 30% oder 40% über bzw. unter der mittleren Soll-Geschwindigkeit liegen kann.

Besonders bevorzugt ist es, wenn die Steuerung über einen Geschwindigkeitsadapter dem ersten Regelkreis überlagert wird. Hierdurch kann die von der Steuerung bewirkte Änderung als modifizierte Führungsgröße der Soll-Drehgeschwindigkeit in dem ersten Regelkreis berücksichtigt werden. Die Drehgeschwindigkeit und der Soll-Volumenstrom werden im Rahmen dieser Betrachtung dabei als zueinander direkt proportional und daher austauschbar angesehen. Wie sich herausgestellt hat, verstärken sich die Einflüsse des ersten Regelkreises mit der Druckvorhersage sowie die Einflüsse der Steuerung dabei gegenseitig, so dass die Regelgüte der Regeleinrichtung überproportional vergrößert werden kann.

Der Fördervorgang einer Peristaltikpumpe umfasst regelmäßig mehr als einen, insbesondere mehr als fünf und besonders bevorzugt mehr als zehn Druckzyklen.

Unter einem anderen Aspekt der Erfindung wird ein Injektionsgerät zur Injektion eines Injektionsmittels mithilfe einer als Rollenpumpe ausgebildeten Peristaltikpumpe in einen tierischen oder menschlichen Körper vorgeschlagen, das zur Regelung der Rollenpumpe eine erfindungsgemäße Regeleinrichtung aufweist. Über Injektionsgeräte mit einer derartigen Regeleinrichtung können für Rollenpumpen gleichmäßige Druckzyklen mit geringen Druckamplituden erzielt werden.

Unter einem wiederum anderen Aspekt der Erfindung wird ein Regelungsverfahren für eine Peristaltikpumpe vorgeschlagen, die einen Quetschschlauch und sich zyklisch bewegende Förderelemente zur Förderung eines in dem Quetschschlauch geführten Mediums während eines Fördervorgangs mit einem kontrollierten Volumenstrom in eine mit dem Quetschschlauch verbundene Ausgabeleitung aufweist, wobei die Förderelemente den Quetschschlauch zyklisch komprimieren, so dass sich in der Ausgabeleitung ein Druckverlauf eines Drucks einstellt, der zyklisch sich wiederholende Druckzyklen aufweist, wobei jeder Druckzyklus ein Druckminimum, einen Druckanstieg, ein Druckmaximum und einen Druckabfall aufweist, wobei das Regelungsverfahren eine Geschwindigkeit der Peristaltikpumpe so regelt, dass ein maximaler Volumenstrom erreicht wird, ohne dabei eine Druckgrenze in der Ausgabeleitung zu überschreiten, wobei das Regelungsverfahren einen ersten Regelkreis zur Regelung des maximalen Volumenstroms umfasst, der als Führungsgrößen einen Soll-Volumenstrom und die Druckgrenze erhält, und für jeden Druckzyklus ein Vorhersagedruck für einen zu erwartenden Maximaldruck innerhalb des Druckzyklus auf Basis des Drucks in der Ausgabeleitung berechnet wird. Der maximale Volumenstrom wird sodann unter Berücksichtigung des Vorhersagedrucks so begrenzt, dass der Druck in der Ausgabeleitung die Druckgrenze in dem Druckzyklus nicht überschreitet.

Weitere Merkmale und vorteilhafte Ausführungen der Erfindung ergeben sich aus den Unteransprüchen und der nachfolgenden Beschreibung, in der die Erfindung anhand von Ausführungsbeispielen unter Verweis auf die beigefügten Figuren beispielhaft beschrieben wird, wobei jeweils gleichermaßen auf die erfindungsgemäße Regeleinrichtung, das erfindungsgemäße Verfahren sowie die erfindungsgemäße Rollenpumpe und das erfindungsgemäße Injektionsgerät Bezug genommen wird.

Dabei zeigen

Fig. 1: eine schematische Wiedergabe eines Kontrastmittel-Injektionsgeräts mit einer Rollenpumpe,

Fig. 2: eine Detaildarstellung einer Rollenpumpe mit Förderelementen,

Fig. 3: ein Diagramm eines typischen, zeitlichen Druckverlaufs einer Rollenpumpe sowie dazugehörige Druckphasen und eines zeitlichen Drehwinkelverlaufs der Förderelemente,

Fig. 4: Ein Diagramm eines typischen Druckverlaufs einer erfindungsgemäß geregelten Rollenpumpe mit einer Phasenerkennung

**Fig. 5:** ein Schaltbild einer erfindungsgemäßen Regeleinrichtung,

**Fig. 6:** ein Druckverlauf bei okkludierter Ausgangsleitung mit Regelantwort der Regeleinrichtung aus Fig. 5,

**Fig. 7A:** ein Diagramm für eine druckphasenabhängige Steuerung eines maximalen Volumenstroms in einer erfindungsgemäßen Regeleinrichtung, sowie

**Fig. 7B:** ein Diagramm von Druckerlauf, Volumenstrom und Winkelgeschwindigkeit für eine Rollenpumpe mit einer Volumenstromsteuerung gemäß Fig. 7A (links) und ohne Volumenstromsteuerung (rechts).

In den Figuren sind gleiche oder vergleichbare Bauteile, Funktionen oder Elemente mit gleichen oder vergleichbaren Bezugszeichen versehen. Bei sich wiederholenden Verwendung von Bezugszeichen wird auf die jeweils vorhergehende Beschreibung verwiesen.

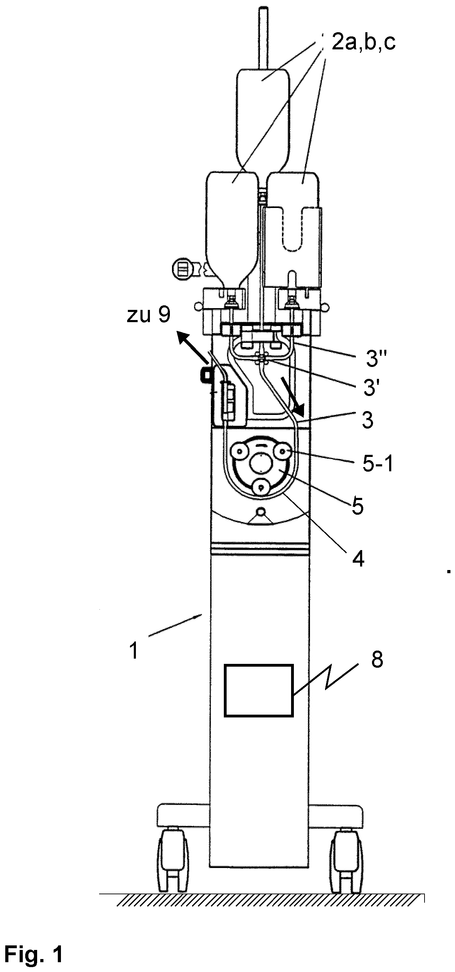

Fig. 1 zeigt ein Injektionsgerät 1 zur intravenösen Verabreichung eines Injektionsmittels. Das Injektionsgerät 1 weist einen auf Rollen gelagerten Grundkorpus auf, an dessen oberem Ende mehrere Injektionsmittelbehälter 2a, 2b, 2c angeordnet sind. In den Injektionsmittelbehältern 2a, 2b, 2c sind Kontrastmittel und eine Kochsalzlösung bevorratet. Die Injektionsmittelbehälter 2a, 2b, 2c sind über eine Zulaufleitung 3 mit einem Quetschlauch 4 verbunden. Der Quetschschlauch 4 ist in das Schlauchbett 6 einer Rollenpumpe 5 eingelegt und ausgangsseitig mit einer Ausgangsleitung 9 verbunden, die wiederum über einen sich daran anschließenden (nicht dargestellten) Patientenschlauch über einen Katheter mit der Blutbahn eines Patienten verbunden werden kann.

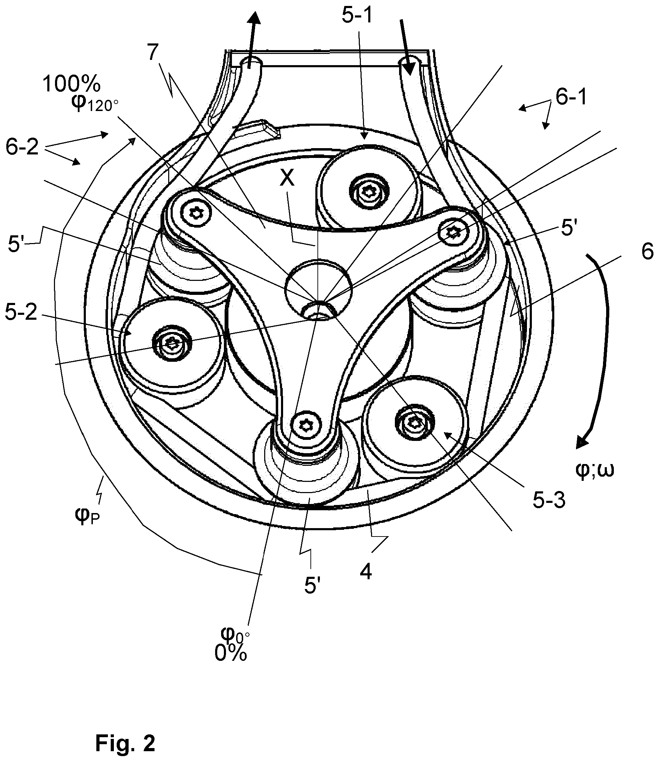

Die Rollenpumpe 5 weist drei an einem elektromotorisch angetriebenen Rotor 7 drehbar gelagerte Rollen 5-1, 5-2, 5-3 auf, die entlang des in Fig. 2 kenntlich gemachten Schlauchbetts 6 der Peristaltikpumpe 5 geführt werden, und dabei den Quetschschlauch 4 bereichsweise gegen ein Gegenlager des Schlauchbetts 6 quetschen, so dass bei Drehung des Rotors 7 um seine Drehachse ein in dem Quetschschlauch 4 befindliches Injektionsmittel portionsweise entgegen einem Staudruck in der Ausgangsleitung 9 in die Ausgangsleitung 9 gefördert wird. Das Injektionsgerät 1 weist eine Regeleinrichtung 8 zur Regelung der Rollenpumpe 5 (und weiterer Komponenten des Injektionsgeräts 1) auf.

In Fig. 2 ist eine perspektivische Detailansicht der Rollenpumpe 5 dargestellt. Wie der Fig. 2 zu entnehmen ist, verläuft der Quetschschlauch 4 über einen Winkelbereich von etwa 260° - bezogen auf die Drehachse X der Rollenpumpe 5 - zwischen einem Einlaufbereich 6-1 des Schlauchbetts 6 und einem Auslaufbereich 6-2 des Schlauchbetts 6. Da die drei Rollen 5-1, 5-2, 5-3 gleichmäßig in einem Winkelabstand von jeweils 120° auf dem Rotor 7 angeordnet sind, befinden sich damit immer wenigstens zwei Rollen zwischen dem Einlaufbereich 6-1 und dem Auslaufbereich 6-2 des Schlauchbetts 6, die den Quetschschlauch 4 gegen das Gegenlager des Schlauchbetts 6 quetschen. Der Quetschschlauch 4 ist daher im Betrieb der Rollenpumpe immer an wenigstens zwei Stellen gequetscht.

Der Rotor 7 der Peristaltikpumpe dreht sich in einer Rotationsrichtung- in der Darstellung von Fig. 2 im Uhrzeigersinn - so dass die gequetschten Bereiche des Quetschschlauchs 4 mit den Rollen 5-1, 5-2, 5-3 in Rotationsrichtung wandern. Zwischen zwei gequetschten Bereichen bildet sich dabei jeweils ein flüssigkeitsdichter Schlauchabschnitt mit einem bestimmten Volumen aus, so dass darin befindliches Injektionsmittel zwischen zwei Rollen 5-1, 5-2, 5-3 eingekapselt in Drehrichtung bis zum Auslaufbereich 6-2 bewegt wird. Wenn im Auslaufbereich 6-2 eine Rolle 5-1 sich von dem Schlauchbett 6 abzuheben beginnt, so kann das Injektionsmittel in dem Schlauchabschnitt entgegen den im restlichen Teil der Ausgangsleitung 9 vorherrschenden Staudruck gefördert werden. In der Fig. 2 ist die Rolle 5-1 in Drehrichtung bereits vollständig aus dem Auslaufbereich 6-2 ausgetreten (der Quetschschlauch 4 wird also nicht mehr durch die Rolle 5-1 gequetscht), und das Injektionsmittel wird in dem Schlauchabschnitt entgegen den im restlichen Teil der Ausgangsleitung 9 vorherrschenden Staudruck gefördert.

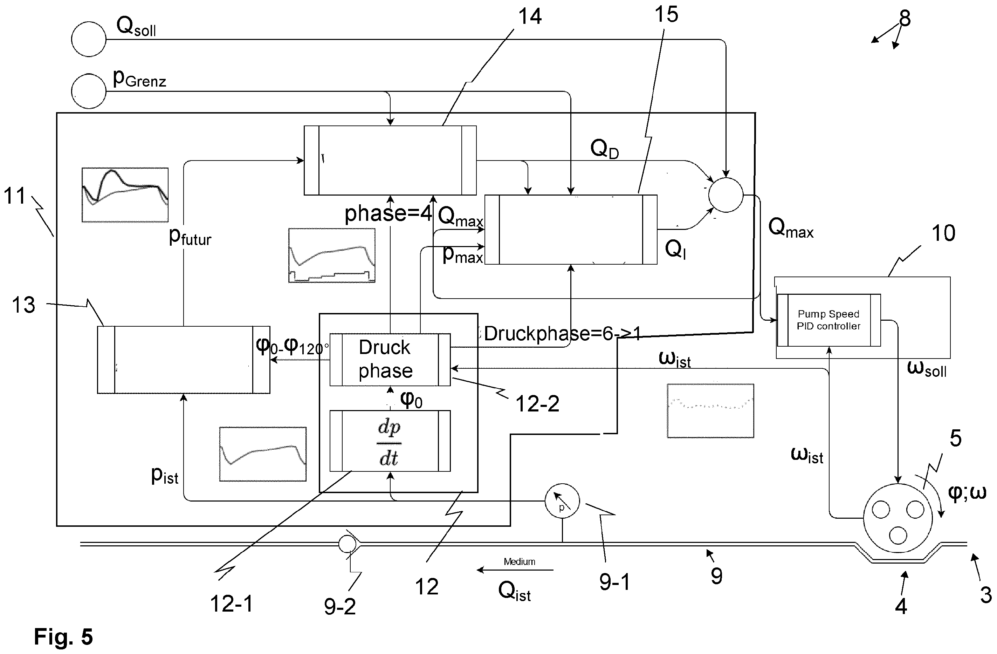

In der Ausgangsleitung 9 ist ein in Fig. 5 dargestellter Drucksensor 9-1 angeordnet, welcher den darin vorherrschenden Druck $p_{ist}$ laufend misst und an die Regeleinrichtung 8 weiterleitet.

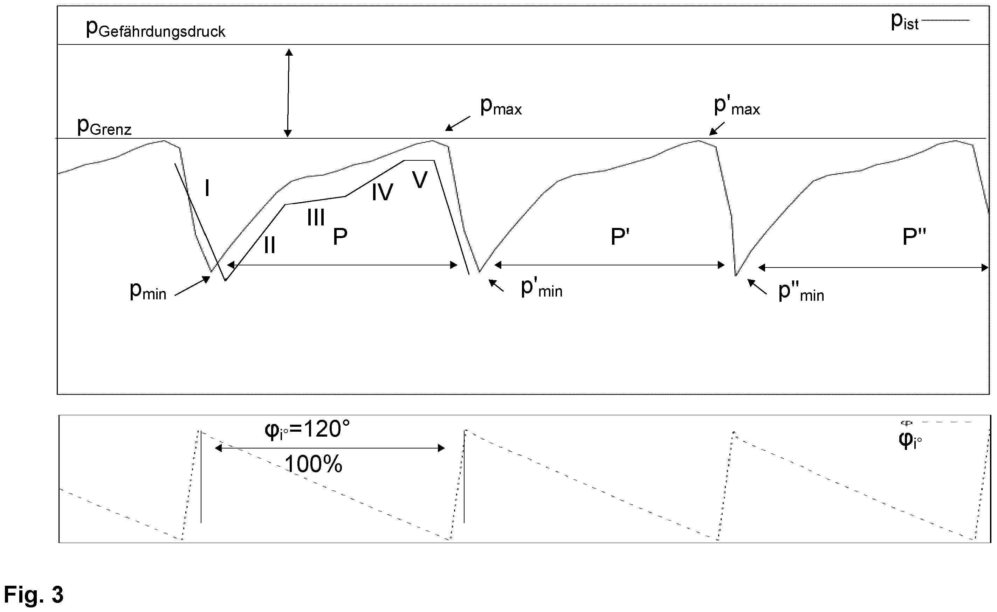

Durch den zyklischen Austritt einer Rolle 5-1 im Auslaufbereich 6-2 ergibt sich in der Ausgangsleitung 9 ein charakteristischer Druckverlauf des Drucks $p_{ist}$ mit zyklisch sich wiederholenden Druckzyklen P. Ein charakteristischer Druckverlauf $p_{ist}$ mit mehreren Druckzyklen P, P', P'' ist exemplarisch in Fig. 3 oben in seinem zeitlichen Verlauf dargestellt. Während eines Druckzyklus P schwankt der Druck zwischen einem Druckminimum $p_{min}$ zu Beginn des Druckzyklus P, steigt zunächst schnell an, geht sodann in einen Bereich mit moderatem Druckanstieg über und erreicht ein kurzzeitiges Druckplateau bzw. Druckmaximum $p_{max}$, bevor der Druck rasch abfällt und der nächste Druckzyklus P' beginnt.

Der Beginn eines Druckzyklus P korreliert dabei mit einem Austritt der ersten Rolle 5-1 aus dem Schlauchbett 6 im Auslaufbereich 6-2, zu welchem Zeitpunkt eine zweite Rolle 5-2 sich in der in Fig. 2 mit dem Bezugszeichen $\varphi_0$ gekennzeichneten Winkelposition befindet. Diese zweite Rolle 5-2 stellt in diesem Augenblick (und bis zum Austritt über den Austrittsbereich 6-2) die führende Rolle dar und verkleinert bei weiterer Drehung das vor Ihr befindliche Schlauchvolumen des Quetschschlauchs 4 entgegen einem Staudruck in der Ausgangsleitung 9 immer weiter, was zu dem charakteristischen Druckanstieg führt. Wenn diese zweite Rolle das Schlauchbett 6 im Auslaufbereich 6-2, d.h. ungefähr bei einer Winkelposition, die in der Fig. 2 mit dem Be-

zugszeichen $\varphi_{120°}$ versehen ist, verlässt, wird die Quetschung des Quetschschlauchs 4 in diesem Bereich aufgehoben, so dass sich das Volumen näherungsweise schlagartig bis zur darauffolgenden dritten Rolle 5-3 vergrößert. Dies führt zu dem charakteristischen Druckabfall am Ende eines Druckzyklus P.

Wie in Fig. 3 ersichtlich, ist der Druckverlauf zu Beginn des Druckzyklus vergleichsweise steil (Bereich II), solange ein in der Ausgangsleitung 9 befindliches Rückschlagventil 9-2 (s. Fig. 5), noch geschlossen ist. Sobald der Schließdruck des Rückschlagventils 9-2 erreicht ist, öffnet das Rückschlagventil 9-2 und der Druckanstieg fällt hierdurch ab (Bereich III). Zu diesem Zeitpunkt muss die Flüssigkeitssäule des Injektionsmittels in der Ausgangsleitung 9 und dem stromabwärtigen Patientenschlauch entgegen der Massenträgheiten sowie weiterer Drosselwiderstände in Bewegung gesetzt werden, was für den weiteren Druckanstieg verantwortlich ist. Ein weiterer Druckanstieg ergibt sich im Bereich IV, der auf ein weiteres Rückschlagventil in einem Katheter zurückzuführen ist. Nachdem die Druckwiderstände überwunden sind, stellt sich ein quasistatischer Strömungszustand mit einem näherungsweise konstanten Druck (Bereich V) ein, bevor der Druck bei Austritt einer Rolle aus dem Schlauchbett 6 wieder abfällt (Bereich I).

Bei einer kompletten Umdrehung des Rotors 7 wird daher jede der drei Rollen 5-1, 5-2, 5-3 einmal den mit $\varphi_P$ bezeichneten Winkelbereich zwischen $\varphi_{0°}$ und $\varphi_{120°}$ durchlaufen und einen Druckzyklus P hervorrufen. Der Winkelbereich $\varphi_P$ umfasst also hier als Druckphasenwinkel bezeichnete Winkel $\varphi_{i°}$ zwischen 0° und 120°, wobei das Subskript i für den Winkel steht. Es ist evident, dass der Druckphasenwinkel $\varphi_{i°}$ dabei unmittelbar mit der Winkelposition $\varphi$ des Rotors 7 bzw. der einzelnen Rollen über die modulo-Beziehung $\varphi_{i°} = \varphi \bmod 120°$ zusammenhängt.

Zur Verdeutlichung ist ein zeitlicher Verlauf des Druckphasenwinkels $\varphi_{i°}$ in Fig. 3 unten eingezeichnet.

Um einen tatsächlichen Volumenstrom $Q_{ist}$ in der Ausgangsleitung 9 möglichst nahe an einem vorgegebenen Soll-Volumenstrom zu führen, ist es notwendig, den tatsächlichen Druckverlauf des Drucks $p_{ist}$ möglichst nahe an der noch zulässigen Druckgrenze $p_{Grenz}$ zu führen. In dem vorliegenden Ausführungsbeispiel der Erfindung soll die Rollenpumpe 5 dabei auf einen maximalen Volumenstrom $Q_{max}$ geregelt werden, ohne dass die noch zulässige Druckgrenze $p_{Grenz}$ überschritten wird. Die Druckgrenze $p_{Grenz}$ kann daher auch als (maximaler) Solldruck für den Druck $p_{ist}$ aufgefasst werden. Oberhalb des Grenzwerts $p_{Grenz}$ ist in Fig. 3 zusätzlich ein Gefährdungsdruck $p_{Gefährdungsdruck}$ eingezeichnet, bei welchem irreparable Schäden an Gerät oder Patient auftreten. Es muss daher sichergestellt sein, dass der Gefährdungsdruck $p_{Gefährdungsdruck}$ auf keinen Fall überschritten wird.

Anders als bei Spritzenpumpen, bei denen ein Kolben mit kontrollierter Geschwindigkeit in einen Zylinder geschoben wird, ist das Einhalten der Druckgrenze $p_{Grenz}$ bei Rollenpumpen aufgrund der Druckpulsationen wesentlich erschwert. Aus diesem Grund müssen bei bekannten Rollenpumpen regelmäßig hohe Sicherheitspuffer vorgehalten werden (Abstand zwischen $p_{Grenz}$ und $p_{Gefährdungsdruck}$), um zu vermeiden, dass bei Überschreiten der Druckgrenze $p_{Grenz}$ bereits zu Beginn eines Druckzyklus der Gefährdungsdruck $p_{Gefährdungsdruck}$ nicht noch im weiteren Verlauf eines bzw. des Druckzyklus erreicht oder sogar überschritten wird.

Um diesen systembedingten Nachteil auszugleichen, sieht die Regeleinrichtung 8 dieses Ausführungsbeispiels eine Regelung gemäß dem in Fig. 5 dargestellten Regelschema vor. Die Regeleinrichtung 8 umfasst einen zweiten Regelkreis 10 zur Regelung der Dreh(winkel)geschwindigkeit $\omega$ des Rotors 7 der Rollenpumpe 5, den Pumpengeschwindigkeitsregler (Pump Speed PID controller), der als Führungsgröße den maximalen Volumenstrom $Q_{max}$ erhält und diesen in eine Soll-Dreh(winkel)geschwindigkeit $\omega_{Soll}$ bzw. ein dazu korrespondierendes Drehfeld zur Ansteuerung der Rollenpumpe umrechnet. Die tatsächliche Ist- Dreh(winkel) geschwindigkeit $\omega_{ist}$ wird als Meßgröße zurückgeführt und eine Regelabweichung zwischen der Soll- und Ist Dreh(winkel)geschwindigkeit per Rückkopplung minimiert.

Zur Bestimmung des maximalen Volumenstrom $Q_{max}$ ist dem zweiten Regelkreis 10 ein erster Regelkreis 11 vorgeschaltet, dem als Führungsgrößen ein Soll-Volumenstrom $Q_{Soll}$ sowie eine zulässige Druckgrenze $p_{Grenz}$ extern vorgegebenen werden. Als Rückführungsgrößen gehen - unverändert - der Soll-Volumenstrom $Q_{Soll}$, der in der Ausgangsleitung 9 gemessene Druck $p_{ist}$ und die an der Rollenpumpe 5 gemessene Drehwinkelgeschwindigkeit $\omega_{ist}$ des Rotors 7 der Rollenpumpe 5 in den ersten Regelkreis 11 ein. Auf die Messung und Rückführung eines Ist-Volumenstroms $Q_{ist}$ in der Ausgangsleitung 9 kann verzichtet werden.

Der erste Regelkreis 11 ist als unstetiger PID-Regler aufgebaut, der die Regelgröße des maximalen Volumenstroms $Q_{max}$ unterschiedlich regelt, je nach dem in welcher Druckphase ein Druckzyklus sich befindet.

Der erste Regelkreis 11 umfasst eine Druckphasenerkennung 12 mit einem Druckphasenkommutierungsdetektor 12-1 und einem Druckphasenschalter 12-2, eine Druckvorhersage 13 sowie einen ersten Regelanteil 14 und einen zweiten Regelanteil 15.

Der Druckphasenkommutierungsdetektor 12-1 dient der Erkennung des Beginns einer Druckphase. Er vergleicht hierzu laufend den aktuellen Druck $p_{ist}$ mit einem Referenzwert, nämlich einem zwischengespeicherten Druckmaximum $p_{max}$ eines vorhergehenden Druckzyklus P, und setzt den Beginn eines Druckzyklus P auf den Zeitpunkt, an dem ein Druckabfall des Drucks $p_{ist}$ in der Ausgabeleitung 9 von mehr als 1/6 gegenüber dem zwischengespeicherten Druckmaximum $p_{max}$ auftritt. Zu diesem Zeitpunkt befindet sich eine der Rollen 5-1, 5-2, 5-3 der Rollenpumpe 5 näherungsweise in der in Fig. 2 mit $\varphi_{0°}$ gekennzeichneten Winkelposition. Der

Druckphasenwinkel $\varphi_{i°}$ wird zu Null gesetzt, d.h. $\varphi_{0°}$=0°, und dient im weiteren Verlauf des Druckzyklus P als Referenzwert für die Bestimmung nachfolgender Druckphasen. Als aktuelle Druckphase wird die "Phase I" gesetzt.

Der Druckphasenschalter 12-2 schaltet bzw. zählt die einzelnen Druckphasen II bis V hoch. Die einzelnen Druckphasen bzw. der Beginn der einzelnen Druckphasen werden über den Winkel $\varphi_{i°}$ bestimmt. Der Druckphasenwinkel $\varphi_{i°}$ wiederum wird - ausgehend von dem Referenzwert $\varphi_{0°}$ über eine zeitliche Integration der Ist-Drehwinkelgeschwindigkeit der Rollenpumpe erfasst bzw. angenähert.

Die unterschiedlichen Druckphasen I bis V sind wie folgt definiert:

Der Beginn der ersten Druckphase I ist über einen Druckabfall von mehr als 1/6 gegenüber dem Druckmaximum des vorhergehenden Druckzyklus definiert. Der Beginn der zweiten Druckphase II wird auf den Zeitpunkt gelegt, an bzw. nachdem ein Druckanstieg sicher erfolgt. Dieser sichere Druckanstieg kann durch eine geeignete Kennzahl des Druckverlaufs festgelegt und daran erkannt werden Die dritte Druckphase III entspricht einer ersten Kompressionsphase, deren Beginn bei einem Druckphasenwinkel von 45° (dies entspricht einem Phasenfortschritt von 37,5% des kompletten Winkelbereichs $\varphi_P$) definiert wird. Eine vierte Druckphase IV wird bei einem Druckphasenwinkel von 60° (Phasenfortschritt von 50%) definiert. Der Beginn der fünften Druckphase V wird bei einem Druckphasenwinkel von 97,2° (Phasenfortschritt von 81%) gesetzt.

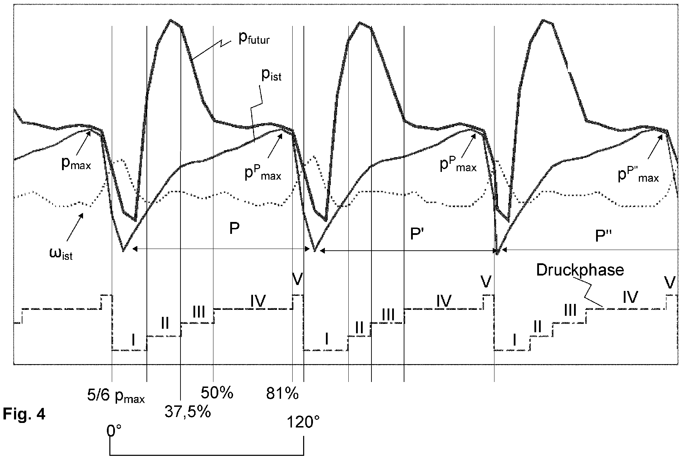

Die einzelnen Druckphasen I bis V von mehreren aufeinanderfolgenden Druckzyklen P, P', P" sind der Fig. 4 entnehmbar.

Über die Druckphasenerkennung 12 wird der erste Regelanteil 14 aktiviert, wenn der aktuelle Druckzyklus P sich in der vierten Druckphase IV befindet. In allen anderen Druckphasen (I bis III und V) ist der erste Regelanteil 14 deaktiviert, d.h. zu Null gesetzt.

Bei dem ersten Regelanteil 14 handelt es sich um das Differentialglied des PID-Reglers. In ihm wird ein Korrekturfaktor $Q_D$ berechnet, der von dem Soll-Volumenstrom $Q_{Soll}$ in Abzug gebracht wird. Er berechnet sich als Verhältnis eines vorhergesagten Drucks $p_{futur}$ (bzw. $p_{forecast}$) und der Druckgrenze $p_{Grenz}$ multipliziert mit dem aktuell berechneten maximalen Volumenstrom $Q_{max}$ nach der Formel

$$Q_D = K_D \frac{p_{futur}}{p_{Grenz}} Q_{max} \,,$$

wobei $K_D$ ein fester Verstärkungsfaktor ist.

Dabei wird der vorhergesagte Druck $p_{futur}$ von der Druckvorhersage 13 laufend zur Verfügung gestellt. Der vorhergesagte Druck $p_{futur}$ wird als lineare Extrapolation des aktuellen Drucks $p_{ist}$ zu einem definierten Druckphasenwinkel von 105° (oder einem Phasenfortschritt von 87,5 %) nach der Formel

$$p_{futur} = p_{ist} \frac{dp_{ist}}{dt} \frac{\varphi t}{\varphi 87{,}5\%}$$ bestimmt, wobei

$\dfrac{dp_{ist}}{dt}$ die zeitliche Ableitung des gemessenen Drucks

$p_{ist}$ und $\dfrac{\varphi}{\varphi 87{,}5\%}$ das Verhältnis des aktuellen Druckphasenwinkels $\varphi_{i=t}$ zum Zeitpunkt t und dem Druckphasenwinkel zu einem Phasenfortschritt von 87,5% (dies entspricht bei einem kompletten Winkelbereich $\varphi_P$ von 120° einem Druckphasenwinkel von 105°) bestimmt. Der Vorhersagedruck stellt eine Prognose für das zu erwartende Druckmaximum in dem aktuellen Druckzyklus zum definierten Druckphasenwinkel dar. Der vorhergesagte Druck $p_{futur}$ ist in seinem zeitlichen Verlauf in Fig. 4 eingezeichnet.

Die Begriffe "Vorhersagedruck" und "vorhergesagter Druck" sind synonym zu verstehen.

Bei dem zweiten Regelanteil 15 handelt es sich um das Proportional- und Integralglied des PID-Reglers. In ihm wird ein Korrekturfaktor $Q_I$ berechnet, der von dem Soll-Volumenstrom $Q_{Soll}$ in Abzug gebracht wird und über drei Komponenten $\alpha$, $\beta$, $\gamma$ berechnet wird:

Die erste Komponente $\alpha$ des zweiten Regelanteils 15 ist ein Mittelwertfilter, das den maximalen Volumenstrom $Q_{max}$ über das Verhältnis des Maximaldrucks $p_{max}$ des unmittelbar vorhergehenden Druckzyklus P gegenüber der Druckgrenze $p_{Grenz}$, multipliziert mit einem Verstärkungsfaktor $K_I$, gemäß der Formel

$$\alpha = K_I \frac{p_{max}}{p_{Grenz}} Q_{max}$$

anpasst. Der maximale Druck $p_{max}$ jedes Druckzyklus wird hierfür in der Regeleinrichtung 8 in einem Speicher zwischengespeichert. Über das Mittelwertfilter können Messfehler des Drucks $p_{ist}$ geglättet werden.

Die zweite Komponente $\beta$ des zweiten Regelanteils 15 ist ein Proportionalglied, das den im vorangegangenen Druckzyklus P von dem ersten Regelanteil 14 berechneten Korrekturfaktor $Q_D$ um einen Verstärkungsfaktor $K_{D \to I}$ gemäß der Formel

$$\beta = K_{D \to I} Q_D$$

verstärkt. Da der erste Regelanteil 14 den Korrekturfaktor $Q_D$ nur in der Druckphase IV berechnet, ist die zweite Komponente $\beta$ des zweiten Regelanteils 15 null, wenn der Druckzyklus sich außerhalb der Druckphase IV befindet.

Die dritte Komponente $\gamma$ des zweiten Regelanteils 15 ist ein Integralglied, das aus der Summe der vorhergehenden Volumenstromkorrekturen $Q_{I\ n-1}$ der einzelnen Druckphasen des aktuellen Druckzyklus, nach der Formel

$$\gamma = \sum_{n-1} Q_{I\,n-1}$$

berechnet wird, wobei n die Anzahl der Druckzyklen und $Q_{I\,n-1}$ die Volumenstromkorrektur von aufeinanderfolgenden Druckzyklen darstellt

Die drei Komponenten $\alpha$, $\beta$, $\gamma$ des zweiten Regelanteils werden zu einer Korrekturgröße $Q_I$ aufaddiert ($Q_I = \alpha + \beta + \gamma$) und die Korrekturgröße $Q_I$ von dem Soll-Volumenstrom $Q_{Soll}$ abgezogen.

Damit ergibt sich der maximale Soll-Volumenstrom $Q_{max}$ aus dem Soll-Volumenstrom $Q_{soll}$ abzüglich der Korrekturgrößen $Q_D$ und $Q_I$ des ersten und des zweiten Regelanteils 14 bzw. 15 ($Q_{max} = Q_{Soll} - Q_I - Q_D$).

Über die so ausgebildete Regeleinrichtung 8 kann die Leistung der Rollenpumpe 5 bzw. deren Drehgeschwindigkeit $\omega$ vorausschauend reguliert und angepasst werden. Da der maximale Volumenstrom $Q_{max}$ in den Druckphasen I bis III und V anders berechnet wird als in der Druckphase IV, handelt es sich um eine nichtstetige Regelung.

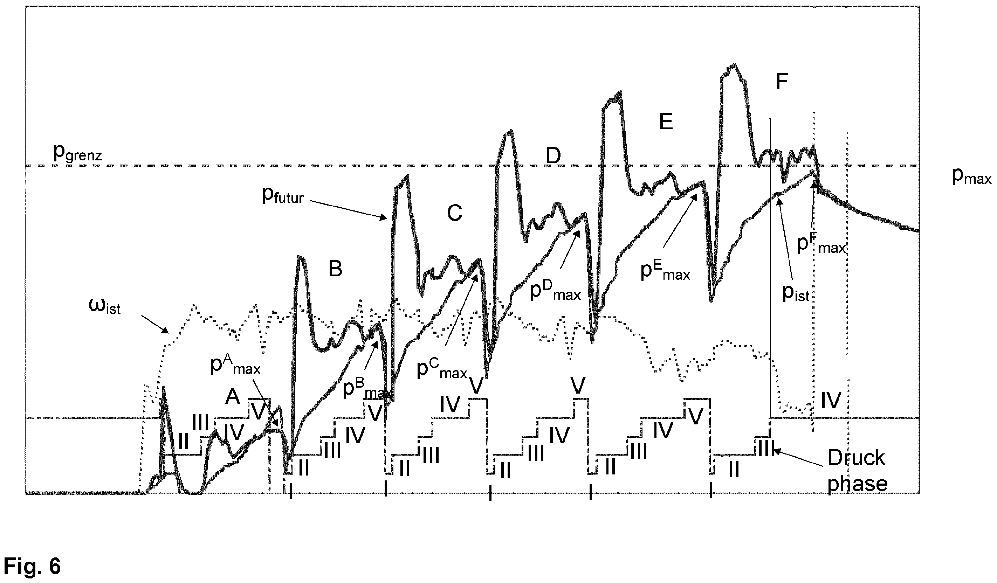

Eine Regelantwort für den Fall einer abgeklemmten, fluidundurchlässigen Ausgangsleitung 9 zeigt das Diagramm der Fig. 6 mit sechs aufeinanderfolgenden Druckzyklen A bis F des gemessenen Drucks $p_{ist}$, den von der Druckvorhersage 13 berechneten Vorhersagedruck $p_{futur}$ und die dazugehörigen Druckphasen I bis V der einzelnen Druckzyklen A bis F. Infolge der abgeklemmten Ausgangsleitung 9 steigt das absolute Druckmaximum $p_{max}$ in jedem Druckzyklus stetig an: $p^A_{max} < p^B_{max} < p^C_{max} < p^D_{max}$ usw.

Der Vorhersagedruck $p_{futur}$ überschreitet dabei die Druckgrenze $p_{Grenz}$ in einer vierten Druckphase IV erstmalig in Druckzyklus F. Die übrigen Überschreitungen des Vorhersagedrucks $p_{futur}$ über die Druckgrenze $p_{Grenz}$ hinaus in anderen Phasen, z.B. in dem Druckzyklus D in der Druckphase II, sind unbeachtlich, da die Regeleinrichtung 8 den Vorhersagedruck $p_{futur}$ nur in der Druckphase IV über den Regelanteil 14 berücksichtigt.

Da jede vierte Druckphase IV bereits bei einem Druckphasenwinkel von 60° (oder einem Phasenfortschritt von 50%) beginnt, wird der maximale Soll-Volumenstrom $Q_{max}$ in dem Druckzyklus F und damit auch die Drehgeschwindigkeit $\omega_{ist}$ mit Einsetzen der Druckphase IV in dem Druckzyklus F von der Regeleinrichtung 8 reduziert. Die Drosselung der Drehgeschwindigkeit $\omega_{ist}$ zu Beginn der Phase IV ist in der Fig. 6 dabei gut zu erkennen. Ein Überschreiten der Druckgrenze $p_{Grenz}$ tritt aufgrund der ausreichenden Vorlaufzeit zwischen Erkennung einer drohenden Überschreitung der Druckgrenze $p_{Grenz}$ und dem Auftreten des Druckmaximums $p^F_{max} < p_{Grenz}$ nicht ein.

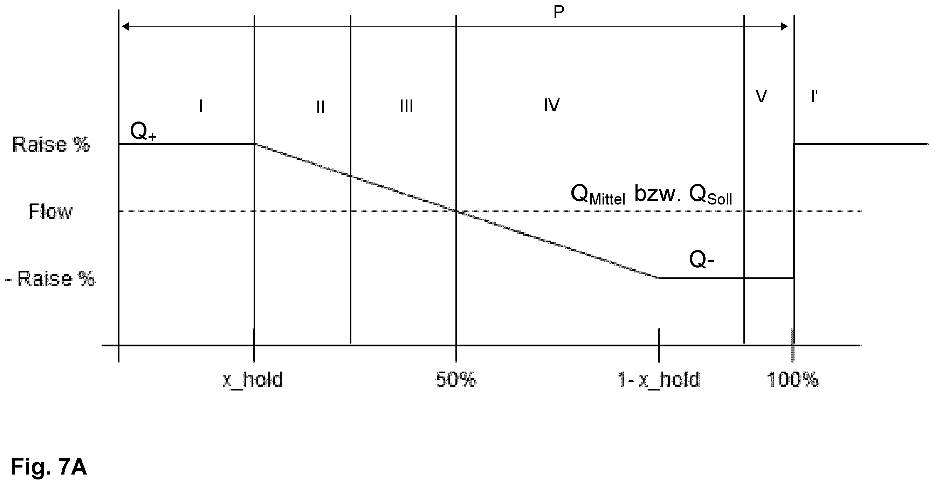

Um eine Nivellierung des Druckverlaufs des Drucks $p_{ist}$ zu erreichen, d.h. eine Reduzierung der Amplitude, kann die Regeleinrichtung 8 des Ausführungsbeispiels nach Fig. 5 außerdem um eine in Fig. 5 nicht dargestellte Steuerung ergänzt werden, die den maximalen Soll-Volumenstrom $Q_{Soll}$ je nach Phasenfortschritt erhöht bzw. erniedrigt. Diese Steuerung manipuliert die Führungsgröße des Soll-Volumenstroms $Q_{Soll}$ dabei so, dass der Soll-Volumenstrom $Q_{Soll}$ zu Beginn eines Druckzyklus um 30% auf einen erhöhten Soll-Volumenstrom Q+ erhöht und gegen Ende eines Druckzyklus um 30% auf einen erniedrigten Soll-Volumenstrom Q_ reduziert wird. Der geänderte Soll-Volumenstrom $Q_{Soll}$ wird dabei jeweils für eine Haltedauer von x-hold, gehalten. Die Haltedauer x-hold kann dabei einem gewissen Phasenfortschritt, wie z.B. 25% oder der Dauer der Phase I, entsprechen. Durch die gegengleiche Anpassung auf den erhöhten bzw. erniedrigten Soll-Volumenstrom Q+ bzw. Q_ bleibt der durchschnittliche Soll-Volumenstrom über einen Druckzyklus P unverändert. Die entsprechende Vorgabe des Soll-Volumenstroms ist in Fig. 7A für einen Druckzyklus P mit den Druckphasen I bis V dargestellt. Wie der Fig. 7A zu entnehmen ist, wird der Soll-Volumenstrom $Q_{Soll}$ dabei zu Beginn des Druckzyklus P schlagartig auf den Volumenstrom $Q_+$ angehoben, und sinkt dann linear auf den erniedrigten Soll-Volumenstrom Q_ ab. Die so veränderte Führungsgröße des Soll-Volumenstroms $Q_{Soll}$, die nun zeitlich über einen Druckzyklus P variabel ist, wird in den ersten Regelkreis 11 eingespeist.

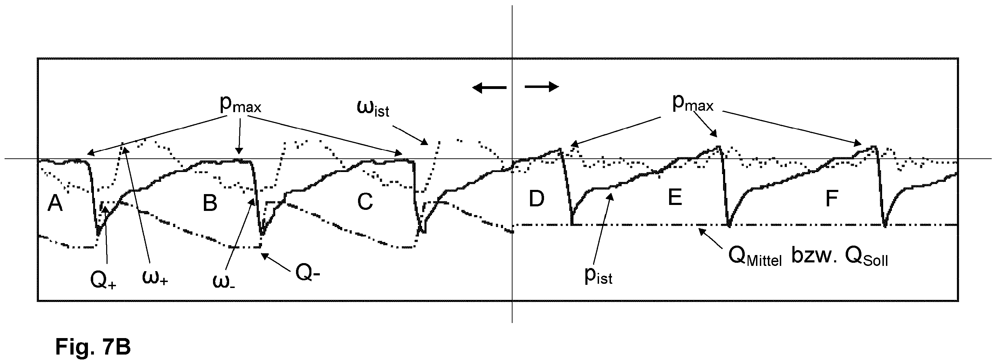

Eine derartige Steuerung hat einen gleichmäßigeren Druckverlauf mit geringeren Druckschwankungen und insbesondere abgeflachten Druckspitzen zur Folge. Dies ist in Fig. 7B dargestellt: In Fig. 7B ist ein Druckverlauf für mehrere Druckzyklen A bis F eingezeichnet, wobei der Soll-Volumenstrom $Q_{Soll}$ in den Druckzyklen A bis C und dem Beginn des Druckzyklus D entsprechend des veränderlichen Soll-Volumenstroms $Q_{Soll}$ gemäß Fig. 7A angepasst wurde, und in den darauffolgenden Druckzyklen ein konstanter Soll-Volumenstrom vorgegeben wurde. Wie der Fig. 7B zu entnehmen, können dadurch die Druckspitzen $p_{max}$ der Druckzyklen A, B und C gegenüber den Druckspitzen $p_{max}$ der Druckzyklen D, E und F verringert werden.

Ein weiterer Effekt dieser Steuerung ist auch, dass eine Motorleistung der Peristaltikpumpe 5 nach dem plötzlichen Druckabfall am Ende eines Druckzyklus P nicht schlagartig reduziert werden oder sogar abgebremst werden muss, um den Volumenstrom konstant zu halten. Dadurch kann Energie eingespart und es können Motorgeräusche reduziert werden.

Da der Soll-Volumenstrom $Q_{Soll}$ näherungsweise direkt proportional zu der Drehwinkelgeschwindigkeit $\omega_{ist}$ der Peristaltikpumpe 5 ist, weist die Drehwinkelgeschwindigkeit $\omega_{ist}$ der Peristaltikpumpe 5 (abzüglich Messungenauigkeiten und dem Einfluss der Regelstrecke) einen vergleichbaren Verlauf wie der Soll-Volumenstrom $Q_{Soll}$ auf. Es ist daher möglich, anstelle des Soll-Volumenstroms auch die Drehwinkelgeschwindigkeit $\omega_{Soll}$ der Peristaltikpumpe druckphasenabhängig auf eine erhöhte Soll-Geschwindigkeit $\omega_+$ zu Beginn eines Druckzyklus zu erhöhen und/oder auf eine erniedrigte Soll-Geschwindigkeit $\omega_-$ gegen Ende des Druckzyklus zu erniedrigen.

Die in den beschriebenen Ausführungsbeispielen exemplarisch dargestellte Erfindung ermöglicht einen sichereren Betrieb einer Peristaltikpumpe bei höherem Mediendurchsatz und geringeren Druckschwankungen.

Die Regeleinrichtung kann neben Peristaltikpumpen auch für andere Pumpen mit zyklisch sich wiederholenden Druckverläufen, wie z.B. Membranpumpen mit einer oszillierenden Membran, Kolbenpumpen mit hin und her bewegten Kolben, Sinuspumpen oder Zahnradpumpen, eingesetzt werden. Die Regeleinrichtung eignet sich insbesondere für Anwendungszwecke, bei denen Überdruckventile, da über diese Medium abgelassen wird, systembedingt ungeeignet sind.

Bezugszeichenliste

| I bis V | Druckphasen eines Druckzyklus |
|---|---|
| $p_{Grenz}$ | Druckgrenze (Soll-Druck) |
| $p_{futur}$ | Vorhersagedruck |
| $p_{Gefährdungsdruck}$ | Gefährdungsdruck |
| P | Druckzyklus (Periode) |
| $Q_{max}$ | maximaler Volumenstrom |
| $Q_{soll}$ | Soll-Volumenstrom |
| X | Drehachse (Rollenpumpe) |

| $\varphi_{i°}$ | Druckphasenwinkel (bei einem Winkel i) |
|---|---|
| $\omega_{ist}$ | Drehwinkelgeschwindigkeit der Rollenpumpe/ der Rollen 5-1,5-2, 5-3 (Ist-Wert) |
| $\omega_{soll}$ | Drehwinkelgeschwindigkeit (Sollwert) |

| 1 | Injektionsgerät |
|---|---|
| 2a-c | Injektionsmittelbehälter |
| 3, 3', 3" | Zulaufleitung |
| 4 | Quetschschlauch |
| 5 | Peristaltikpumpe (Rollenpumpe) |
| 5-1 | Rolle (Quetschrolle) |
| 5-2 | Rolle (Quetschrolle) |
| 5-3 | Rolle (Quetschrolle) |
| 5' | Führungsrolle |
| 6 | Schlauchbett |
| 6-1 | Einlaufbereich |
| 6-2 | Auslaufbereich |
| 7 | Rotor |
| 8 | Regeleinrichtung |
| 9 | Ausgangsleitung |
| 9-1 | Drucksensor (Injektionsleitung) |
| 9-2 | Rückschlagventil (Injektionsleitung) |
| 10 | zweiter Regelkreis (Pumpengeschwindigkeitskontroller) |
| 11 | erster Regelkreis |
| 12 | Druckphasenerkennung |
| 12-1 | Druckphasenkommutierer |
| 12-2 | Druckphasenschalter |
| 13 | Druckvorhersage |
| 14 | erster Regenanteil |
| 15 | zweiter Regenanteil |

**Patentansprüche**

1. Regeleinrichtung (8) für eine Peristaltikpumpe (5) mit einem Quetschschlauch (4) und sich zyklisch bewegenden Förderelementen (5-1, 5-2, 5-3) zur Förderung eines in dem Quetschschlauch (4) geführten Mediums während eines Fördervorgangs mit einem kontrollierten Volumenstrom in eine mit dem Quetschschlauch (4) verbundene Ausgabeleitung (9), wobei die Förderelemente (5-1, 5-2, 5-3) den Quetschschlauch (4) zyklisch komprimieren, so dass sich in der Ausgabeleitung (9) ein Druckverlauf eines Drucks ($p_{ist}$) einstellt, der zyklisch sich wiederholende Druckzyklen (P) aufweist, wobei jeder Druckzyklus (P) ein Druckminimum, einen Druckanstieg, ein Druckmaximum und einen Druckabfall aufweist, wobei die Regeleinrichtung (8) eine Geschwindigkeit der Peristaltikpumpe (5) so regelt, dass ein maximaler Volumenstrom ($Q_{max}$) erreicht wird, ohne dabei eine Druckgrenze ($p_{Grenz}$) in der Ausgabeleitung (9) zu überschreiten, wobei die Regeleinrichtung (8) einen ersten Regelkreis (11) zur Regelung des maximalen Volumenstroms ($Q_{max}$) aufweist, der als Führungsgrößen einen Soll-Volumenstrom ($Q_{soll}$) und die Druckgrenze ($p_{Grenz}$) erhält, und zur Ausführung eines Regelverfahrens eingerichtet ist, das **dadurch gekennzeichnet ist, dass**

   für jeden Druckzyklus (P) ein Vorhersagedruck ($p_{futur}$) für einen zu erwartenden Maximaldruck innerhalb des Druckzyklus (P) auf Basis mindestens des Drucks ($p_{ist}$) in der Ausgabeleitung (9) berechnet wird, und der maximale Volumenstrom ($Q_{max}$) unter Berücksichtigung des Vorhersagedrucks ($p_{futur}$) so begrenzt wird, dass der Druck ($p_{ist}$) in der Ausgabeleitung (9) die Druckgrenze ($p_{Grenz}$) in dem Druckzyklus (P) nicht überschreitet.

2. Regeleinrichtung (8) nach Anspruch 1, **dadurch gekennzeichnet, dass** der erste Regelkreis (11) eine Druckphasenerkennung (12) umfasst, die das Ende eines vorangehenden Druckzyklus (P) und den Beginn eines darauffolgenden Druckzyklus (P') anhand einer charakteristischen Größe, insbesondere einem definierten Druckabfall gegenüber einem Druckmaximum ($p_{max}$) des vorhergehenden Druckzyklus (P), erkennt, um die Regelung des maximalen Volumenstroms ($Q_{max}$) für den darauffolgenden Druckzyklus (P) einzuleiten.

3. Regeleinrichtung (8) nach einem der vorhergehenden Ansprüche, **dadurch gekennzeichnet, dass** jeder Druckzyklus (P) anhand vordefinierter Charakteristika, insb. einer Druckänderung und/oder einer Druckänderungsrate des Drucks ($p_{ist}$), in sukzessive aufeinanderfolgende Druckphasen (I bis V) eingeteilt ist, und die Druckphasenerkennung (12) den Beginn der einzelnen Druckphasen (I bis V) entweder anhand der vordefinierten Charakteristika, und insb. den Beginn einer Druckphase anhand einer Druckänderung ($\Delta p$) des Drucks ($p_{ist}$) und/oder einer Druckänderungsrate (dp/dt) des Drucks ($p_{ist}$), und/oder anhand einer Position der Förderelemente (5-1, 5-2, 5-3) der Peristaltikpumpe (5) erkennt, und der Vorhersagedruck ($p_{futur}$) zur Regelung des maximalen Volumenstroms ($Q_{max}$) in dem ersten Regelkreis (11) nur in bestimmten Druckphasen verwendet und in anderen Druckphasen ignoriert bzw. nicht verwendet wird.

4. Regeleinrichtung (8) nach Anspruch 3, wobei eine erste Druckphase (I) durch einen schnellen Druckverlust, eine zweite Druckphase (II) durch einen schnellen Druckanstieg, eine dritte Druckphase (III) durch einen flachen Druckanstieg, eine vierte Druckphase (IV) durch einen moderaten Druckanstieg (IV) und eine fünfte Druckphase (V) durch ein Druckplateau mit im Wesentlichen konstantem Druck gekennzeichnet ist, und jede Druckphase durch die Druckphasenerkennung (12) erkannt wird.

5. Regeleinrichtung (8) nach Anspruch 4, **dadurch gekennzeichnet, dass** im ersten Regelkreis (11) der Vorhersagedruck ($p_{futur}$) zur Begrenzung des maximalen Volumenstroms ($Q_{max}$) nur in der vierten Druckphase (IV) verwendet wird, wobei die vierte Druckphase (IV) sich vorzugs- und näherungsweise in einem Bereich des Druckzyklus (P) von 50% bis 80% eines Phasenfortschritts des Druckzyklus (P) bewegt.

6. Regeleinrichtung (8) nach einem der Ansprüche 2 bis 5, **dadurch gekennzeichnet, dass** in dem Regelverfahren der Vorhersagedruck ($p_{futur}$) als vorzugsweise lineare Extrapolation des aktuellen Druckanstiegs ($p_{ist}$) auf einen bestimmten, rechnerischen Phasenfortschritt ($\varphi_{87\%}$) des Druckzyklus (P) bestimmt wird.

7. Regeleinrichtung (8) nach Anspruch 6, **dadurch gekennzeichnet, dass** in dem Regelverfahren der bestimmte, rechnerische Phasenfortschritt ($\varphi_{87\%}$), zu dem der Vorhersagedruck ($p_{futur}$) bestimmt wird, nicht mit einem tatsächlichen Phasenfortschritt ($\varphi_{81\%}$) übereinstimmt, zu dem der tatsächliche Maximaldruck ($p_{max}$) eines Druckzyklus (P) auftritt.

8. Regeleinrichtung (8) nach einem der vorhergehende Ansprüche, **dadurch gekennzeichnet, dass** das der erste Regelkreis (11) einen Mittelwert-Filter umfasst, das den maximalen Volumenstrom ($Q_{max}$) in

Abhängigkeit eines in der Regeleinrichtung (8) zwischengespeicherten Maximaldrucks ($p_{max}$) eines dem aktuellen Druckzyklus (P) unmittelbar vorhergehenden Druckzyklus (P) anpasst.

9. Regeleinrichtung (8) nach einem der vorhergehenden Ansprüche 3 bis 8, **dadurch gekennzeichnet, dass** der erste Regelkreis (11) ein PID-Regler mit einem Proportional-Glied (P), einem Integral-Glied (I) und einem Differential-Glied (D) ist, wobei vorzugsweise das Differential-Glied in bestimmten Druckphasen, insbesondere in der ersten Druckphase (I), der zweiten Druckphase (II), der dritten Druckphase (III) und der fünften Druckphase (V), zu Null gesetzt wird.

10. Regeleinrichtung (8) nach einem der vorhergehende Ansprüche, **dadurch gekennzeichnet, dass** die Regeleinrichtung (8) eine Steuerung zur Glättung von Druckspitzen des Drucks ($p_{ist}$) in den einzelnen Druckzyklen (P) umfasst, wobei diese Steuerung als Geschwindigkeitsadapter eingerichtet ist, durch den jeweils zu einem bestimmten Fortschritt des Druckzyklus eine Soll-Geschwindigkeit ($\omega_{Soll}$) eines Förderelements (5-1, 5-2, 5-3) der Peristaltikpumpe (5) gegenüber einer durchschnittlichen Soll-Geschwindigkeit vor Abschluss eines Druckzyklus (P) um einen bestimmten Betrag auf eine erniedrigte Soll-Geschwindigkeit ($\omega_-$) abgesenkt und/oder nach Abschluss eines Druckzyklus (P) um einen bestimmten Betrag auf eine erhöhte Soll-Geschwindigkeit ($\omega_+$) erhöht und für jeweils eine bestimmte Haltedauer gehalten wird, insbesondere derart, dass die Geschwindigkeit ($\omega$) eines Förderelements (5-1, 5-2, 5-3) nach Abschluss eines Druckzyklus (P) sprungartig um den definierten Betrag erhöht wird und anschließend linear bis auf die erniedrigte Soll-Geschwindigkeit ($\omega_-$) des Förderelements (5-1, 5-2, 5-3) abgesenkt wird.

11. Regeleinrichtung (8) nach einem der vorhergehenden Ansprüche, **dadurch gekennzeichnet, dass** der Fördervorgang mehr als einen, insbesondere mehr als 5 und besonders bevorzugt mehr als 10 Druckzyklen umfasst.

12. Peristaltikpumpe in Form einer Rollenpumpe (5) mit einem drehbaren Rotor (7) und einer Regeleinrichtung (8) nach einem der vorhergehenden Ansprüche,

wobei die Förderelemente (5-1, 5-2, 5-3) zum Quetschen eines Quetschschlauchs (4) als an einem Rotor (7) angeordnete Rollen ausgebildet sind, wobei der Quetschschlauch (4) entlang eines Schlauchbetts (6) mit einem Einlaufbereich (6-1) und einem Auslaufbereich (6-2) gelagert ist und die Förderelemente (5-1, 5-2, 5-3) bei Drehung des Rotors (7) nacheinander jeweils einen Abschnitt des Quetschschlauchs (4) beim Eintreten der Förderelemente (5-1, 5-2, 5-3) in das Schlauchbett (6) auf Höhe des Einlaufbereichs (6-1) und bis zum Austreten der Förderelemente (5-1, 5-2, 5-3) aus dem Schlauchbett (6) auf Höhe des Auslaufbereichs (6-2) fluiddicht quetschen und dadurch das in dem Quetschschlauch (4) befindliche Medium in Drehrichtung entgegen einem Staudruck in der Ausgabeleitung (9) in die Ausgabeleitung (9) fördern,

wobei jeweils ein vor dem Auslaufbereich (6-2) befindliches Volumen des Quetschschlauchs (4) von einem maximalen Volumen auf ein minimales Volumen komprimiert wird, solange bis ein Förderelement (5-1, 5-2, 5-3) auf Höhe des Auslaufbereichs (6-2) aus dem Schlauchbett (6) austritt und dabei den durch dieses Förderelement (5-1, 5-2, 5-3) gequetschten Abschnitt des Quetschschlauchs (4) freigibt und damit das vor dem Auslaufbereich (6-2) befindliche Volumen des Quetschschlauchs (4) von dem minimalen Volumen auf das maximale Volumen vergrößert wird, wobei jeweils eine Volumenreduktion und eine anschließende Volumenvergrößerung des vor dem Auslaufbereich (6-2) befindlichen Volumens zusammen einen vollständigen Druckzyklus (P) ausbilden,

und die Rollenpumpe (5) n vorzugsweise radial gleichverteilte Förderelemente (5-1, 5-2, 5-3) aufweist, so dass die Rollenpumpe (5) bei einer vollständigen Drehung des Rotors (7) um 360° n Pumpenhübe mit n Druckzyklen (P) ausführt, und jede Druckphase (I bis V) eines Druckzyklus (P) im Wesentlichen einem bestimmten Winkelbereich einer Winkelposition ($\varphi$) eines Förderelements (5-1, 5-2, 5-3) entspricht.

13. Peristaltikpumpe nach Anspruch 12, **dadurch gekennzeichnet, dass** durch die Druckphasenerkennung (12) der Beginn mindestens einer Druckphase (I bis V) eines Druckzyklus (P) indirekt über eine Winkelposition ($\varphi$) eines Förderelements (5-1, 5-2, 5-3) bestimmt wird.

14. Injektionsgerät (1) zur Injektion eines Injektionsmittels in einen tierischen oder menschlichen Körper mittels einer als Rollenpumpe (5) ausgebildeten Peristaltikpumpe, **dadurch gekennzeichnet, dass** das Injektionsgerät (1) eine Peristaltikpumpe (5) nach einem der Ansprüche 12 oder 13 enthält.

15. Regelungsverfahren für eine Peristaltikpumpe (5) mit einem Quetschschlauch (4) und sich zyklisch bewegenden Förderelementen (5-1, 5-2, 5-3) zur Förderung eines in dem Quetschschlauch (4) geführten Mediums während eines Fördervorgangs mit einem kontrollierten Volumenstrom in eine mit dem Quetschschlauch (4) verbundene Ausgabeleitung (9), wobei die Förderelemente (5-1, 5-2, 5-3) den Quetschschlauch (4) zyklisch komprimieren, so dass sich in der Ausgabeleitung (9) ein Druckverlauf eines Drucks ($p_{ist}$) einstellt, der zyklisch sich wiederholende Druckzyklen (P) aufweist, wobei jeder Druckzyklus ein Druckminimum, einen Druckanstieg, ein Druckmaximum und einen Druckabfall aufweist, wobei das Regelungsverfahren eine Geschwindigkeit der Peristaltikpumpe (5) so regelt, dass ein maximaler Volumenstrom ($Q_{max}$) erreicht wird, ohne dabei eine Druckgrenze ($p_{Grenz}$) in der Ausgabeleitung (9) zu überschreiten, wobei das Regelungsverfahren einen ersten Regelkreis (11) zur Regelung des maximalen Volumenstroms ($Q_{max}$) umfasst, der als Führungsgrößen einen Soll-Volumenstrom ($Q_{soll}$) und die Druckgrenze ($p_{Grenz}$) erhält, wobei das Verfahren **dadurch gekennzeichnet ist, dass**

für jeden Druckzyklus (P) ein Vorhersagedruck ($p_{futur}$) für einen zu erwartenden Maximaldruck ($p_{max}$) innerhalb des Druckzyklus (P) auf Basis des Drucks ($p_{ist}$) in der Ausgabeleitung (9) berechnet wird und der maximale Volumenstrom ($Q_{max}$) unter Berücksichtigung des Vorhersagedrucks ($p_{futur}$) so begrenzt wird, dass der Druck ($p_{ist}$) in der Ausgabeleitung die Druckgrenze ($p_{Grenz}$) in dem Druckzyklus (P) nicht überschreitet.

## Claims

1. Regulating device (8) for a peristaltic pump (5) with a squeeze hose (4) and cyclically moving conveying elements (5-1, 5-2, 5-3) for conveying a medium guided in the squeeze hose (4) during a conveying process with a controlled volume flow into an output line (9) connected to the squeeze hose (4), wherein conveying elements (5-1, 5-2, 5-3) are cyclically compressing the squeeze hose (4), so that in the output line (9) a pressure profile of a pressure ($p_{ist}$) is produced, which is comprising cyclically repeating pressure cycles (P), wherein each pressure cycle (P) is comprising pressure minimum, a pressure increase, a pressure maximum and a pressure drop, wherein the regulating device (8) controls a speed of the peristaltic pump (5) such that a maximum volume flow ($Q_{max}$) is achieved without exceeding a pressure limit ($p_{Grenz}$) in the output line (9), wherein the regulating device (8) has a first control circuit (11) for controlling the maximum volume flow ($Q_{max}$), which receives a setpoint volume flow ($Q_{soll}$) and the pressure limit ($p_{Grenz}$) as reference variables, and is set up to execute a control method which is **characterised in that**

for each pressure cycle (P), a predicted pressure ($p_{futur}$) is calculated for a maximum pressure to be expected within the pressure cycle (P) on the basis of at least the pressure ($p_{ist}$) in the output line (9), and the maximum volume flow ($Q_{max}$) is limited, taking into account the predicted pressure ($p_{futur}$), in such a way that the pressure ($p_{ist}$) in the output line (9) does not exceed the pressure limit ($p_{Grenz}$) in the pressure cycle (P).

2. Regulating device (8) according to claim 1, **characterised in that** the first control circuit (11) comprises a pressure phase detection (12) which detects the end of a preceding pressure cycle (P) and the beginning of a subsequent pressure cycle (P') on the basis of a characteristic variable, in particular a defined pressure drop compared to a pressure maximum ($p_{max}$) of the preceding pressure cycle (P), in order to initiate the control of the maximum volume flow ($Q_{max}$) for the subsequent pressure cycle (P).

3. Regulating device (8) according to any one of the preceding claims, **characterised in that** each pressure cycle (P) is divided into successive pressure phases (I to V) on the basis of predefined characteristics, in particular a pressure change and/or a pressure change rate of the pressure ($p_{ist}$), and the pressure phase detection (12) determines the start of the individual pressure phases (I to V) either on the basis of the predefined characteristics, and in particular the start of a pressure phase on the basis of a pressure change ($\Delta p$) of the pressure ($p_{ist}$) and/or a pressure change rate (dp/dt) of the pressure ($p_{ist}$), and/or on the basis of a position of the conveying elements (5-1, 5-2, 5-3) of the peristaltic pump (5), and the predicted pressure ($p_{futur}$) is used to control the maximum volume flow ($Q_{max}$) in the first control circuit (11) only in certain pressure phases and is ignored or not used in other pressure phases.

4. Regulating device (8) according to claim 3, wherein a first pressure phase (I) is **characterised by** a rapid pressure drop, a second pressure phase (II) by a rapid pressure rise, a third pressure phase (III) by a flat pressure rise, a fourth pressure phase (IV) by a moderate pressure rise (IV) and a fifth pressure phase (V) by a pressure plateau with substantially constant pressure, and each pressure phase is detected by the pressure phase detection (12).

5. Regulating device (8) according to claim 4, **characterised in that** in the first control circuit (11), the prediction pressure ($p_{futur}$) is used to limit the maximum volume flow ($Q_{max}$) only in the fourth pressure phase (IV), wherein the fourth pressure phase (IV) preferably and approximately is in a range of the pressure cycle (P) of 50% to 80% of a phase progression of the pressure cycle (P).

6. Regulating device (8) according to any one of claims 2 to 5, **characterised in that** in the control method the predicted pressure ($p_{futur}$) is determined as a preferably linear extrapolation of the current pressure increase ($p_{ist}$) to a specific, calculated phase progress ($\varphi_{87\%}$) of the pressure cycle (P).

7. Regulating device (8) according to claim 6, **characterised in that** in the control method the determined, calculated phase progress ($\varphi_{87\%}$), at which the predicted pressure ($p_{futur}$) is determined, does not correspond to an actual phase progress ($\varphi_{81\%}$), at which the actual maximum pressure ($p_{max}$) of a pressure cycle (P) occurs.

8. Regulating device (8) according to any one of the preceding claims, **characterised in that** the first control circuit (11) comprises a mean value filter, which adjusts the maximum volume flow ($Q_{max}$) as a function of a maximum pressure ($p_{max}$), which is temporarily stored in the regulating device (8), of a pressure cycle (P) immediately preceding the current pressure cycle (P).

9. Regulating device (8) according to any one of the preceding claims 3 to 8, **characterised in that** the first control circuit (11) is a PID controller with a proportional element (P), an integral element (I) and a differential element (D), wherein preferably the differential element is set to zero in certain pressure phases, in particular in the first pressure phase (I), the second pressure phase (II), the third pressure phase (III) and the fifth pressure phase (V).

10. Regulating device (8) according to any one of the preceding claims, **characterised in that** the regulating device (8) comprises a controller for smoothing pressure peaks of the pressure ($p_{ist}$) in the individual pressure cycles (P), this controller being set up as a speed adapter, by means of which a setpoint speed ($\omega_{Soll}$) of a conveying element (5-1, 5-2, 5-3) of the peristaltic pump (5) is lowered by a specific amount to a lowered setpoint speed ($\omega-$) compared with an average setpoint speed before completion of a pressure cycle (P) and/or increased by a specific amount to an increased setpoint speed ($\omega_+$) after completion of a pressure cycle (P), and held for a specific holding time in each case at a specific progress of the pressure cycle, in particular in such a way that the speed ($\omega$) of a conveying element (5-1, 5-2, 5-3) is increased abruptly by the defined amount after completion of a pressure cycle (P) and is then lowered linearly to the lowered target speed ($\omega-$) of the conveying element (5-1, 5-2, 5-3).

11. Regulating device (8) according to any one of the preceding claims, **characterised in that** the conveying process comprises more than one, in particular more than 5 and particularly preferably more than 10 pressure cycles.

**12.** Peristaltic pump in the form of a roller pump (5) with a rotatable rotor (7) and a regulating device (8) according to one of the preceding claims,

wherein the conveying elements (5-1, 5-2, 5-3) for squeezing a squeezing hose (4) are designed as rollers arranged on a rotor (7), wherein the squeezing hose (4) is mounted along a hose bed (6) with an inlet region (6-1) and an outlet region (6-2), and the conveying elements (5-1, 5-2, 5-3), upon rotation of the rotor (7), successively squeeze a section of the squeezing hose (4) into the hose bed (6) as the conveying elements (5-1, 5-2, 5-3) enter the hose bed (6), at the level of the inlet region (6-1) and until the conveying elements (5-1, 5-2, 5-3) emerge from the hose bed (6) at the level of the outlet region (6-2) and thereby convey the medium contained in the squeeze hose (4) in the direction of rotation into the output line (9) against a back pressure in the output line (9),

wherein a volume of the squeeze hose (4) located upstream of the outlet region (6-2) is compressed from a maximum volume to a minimum volume until a conveying element (5-1, 5-2, 5-3) emerges from the hose bed (6) at the level of the outlet region (6-2) and in the process squeezes the section squeezed by this conveying element (5-1, 5-2, 5-1, 5-2, 5-3), thereby releasing the section of the squeeze hose (4) squeezed by this conveying element (5-1, 5-2, 5-3) and thereby increasing the volume of the squeeze hose (4) located upstream of the outlet region (6-2) from the minimum volume to the maximum volume, wherein a volume reduction and a subsequent volume increase of the volume located upstream of the outlet region (6-2) together form a complete pressure cycle (P),

and the roller pump (5) has n preferably radially equally distributed conveying elements (5-1, 5-2, 5-3), so that the roller pump (5) performs n pump strokes with n pressure cycles (P) during a complete rotation of the rotor (7) by 360°,

and each pressure phase (I to V) of a pressure cycle (P) essentially corresponds to a specific angular range of an angular position ($\varphi$) of a conveying element (5-1, 5-2, 5-3).

**13.** Peristaltic pump according to claim 12, **characterised in that** the start of at least one pressure phase (I to V) of a pressure cycle (P) is determined indirectly via an angular position ($\varphi$) of a conveying element (5-1, 5-2, 5-3) by the pressure phase detection (12).

**14.** Injection device (1) for injecting an injection agent into an animal or human body by means of a peristaltic pump designed as a roller pump (5), **characterised in that** the injection device (1) contains a peristaltic pump (5) according to one of claims 12 or 13.

**15.** Control method for a peristaltic pump (5) with a squeeze hose (4) and cyclically moving conveying elements (5-1, 5-2, 5-3) for conveying a medium guided in the squeeze hose (4) during a conveying process with a controlled volume flow into an output line (9) connected to the squeeze hose (4), wherein the conveying elements (5-1, 5-2, 5-3) cyclically compress the squeeze hose (4), so that a pressure profile of a pressure ($p_{ist}$) is established in the output line (9), which has cyclically repeating pressure cycles (P), wherein each pressure cycle has a pressure minimum, a pressure increase, a pressure maximum and a pressure drop, wherein the control method controls a speed of the peristaltic pump (5) such that a maximum volume flow ($Q_{max}$) is achieved without exceeding a pressure limit ($p_{Grenz}$) in the output line (9), wherein the control method comprises a first control circuit (11) for controlling the maximum volumetric flow ($Q_{max}$), which receives a target volumetric flow ($Q_{soll}$) and the pressure limit ($p_{Grenz}$) as reference variables, wherein the method is **characterised in that**

for each pressure cycle (P), a predicted pressure ($p_{futur}$) for an expected maximum pressure ($p_{max}$) within the pressure cycle (P) is calculated on the basis of the pressure ($p_{ist}$) in the output line (9), and the maximum volume flow ($Q_{max}$) is limited, taking into account the predicted pressure ($p_{futur}$), in such a way that the pressure ($p_{ist}$) in the output line does not exceed the pressure limit ($p_{Grenz}$) in the pressure cycle (P).

**Revendications**

**1.** Dispositif de régulation (8) pour une pompe péristaltique (5) avec un tuyau à écraser (4) et des éléments d'acheminement (5-1, 5-2, 5-3) se déplaçant de façon cyclique pour l'acheminement d'un milieu guidé dans le tuyau à écraser (4) pendant un processus d'acheminement avec un débit volumétrique commandé jusque dans une conduite de distribution (9) reliée au tuyau à écraser (4), dans lequel les éléments d'acheminement (5-1, 5-2, 5-3) compriment le tuyau à écraser (4) de façon cyclique de sorte qu'un tracé de pression d'une pression ($p_{ist}$) se règle dans la conduite de distribution (9), lequel présente des cycles de pression (P) se renouvelant de façon cyclique, dans lequel chaque cycle de pression (P) présente un minimum de pression, une augmentation de pression, un maximum de pression et une baisse de pression, dans lequel le dispositif de régulation (8) régule une vitesse de la pompe péristaltique (5) de sorte qu'un débit volumétrique maximal ($Q_{max}$) soit atteint sans ce faisant dépasser une limite de pression ($p_{Grenz}$) dans la conduite de distribution (9), dans lequel le dispositif de régulation (8) présente un premier circuit de régulation (11) pour la régulation du débit volumétrique maximal ($Q_{max}$) qui obtient un débit volumétrique de consigne ($Q_{soll}$) et la limite de pression ($p_{Grenz}$) en tant que grandeurs de guidage, et est configuré pour l'exécution d'un procédé de régulation, qui est **caractérisé en ce que**

pour chaque cycle de pression (P) est calculée une pression anticipée ($p_{futur}$) pour une pression maximale à escompter à l'intérieur du cycle de pression (P) sur la base d'au moins la pression ($p_{ist}$) dans la conduite de distribution (9), et le débit volumétrique maximal ($Q_{max}$) est délimité en tenant compte de la pression anticipée ($p_{futur}$) de sorte que la pression ($p_{ist}$) dans la conduite de distribution (9) ne dépasse pas la limite de pression ($p_{Grenz}$) dans le cycle de pression (P).

2. Dispositif de régulation (8) selon la revendication 1, **caractérisé en ce que** le premier circuit de régulation (11) comprend une détection de phase de pression (12) qui détecte la fin d'un cycle de pression précédent (P) et le début d'un cycle de pression suivant (P') à l'aide d'une grandeur caractéristique, en particulier d'une baisse de pression définie par rapport à un maximum de pression ($p_{max}$) du cycle de pression précédent (P), pour lancer la régulation du débit volumétrique maximal ($Q_{max}$) pour le cycle de pression suivant (P).

3. Dispositif de régulation (8) selon l'une des revendications précédentes, **caractérisé en ce que** chaque cycle de pression (P) est divisé en phases de pression (I à V) se suivant de façon successive à l'aide de caractéristiques prédéfinies, en particulier d'une modification de pression et/ou d'un taux de modification de pression de la pression ($p_{ist}$), et la détection de phase de pression (12) détecte le début de chacune des phases de pression (I à V) soit à l'aide des caractéristiques prédéfinies, et en particulier le début d'une phase de pression à l'aide d'une modification de pression ($\Delta p$) de la pression ($p_{ist}$) et/ou d'un taux de modification de pression (dp/dt) de la pression ($p_{ist}$), et/ou soit à l'aide d'une position des éléments d'acheminement (5-1, 5-2, 5-3) de la pompe péristaltique (5), et la pression anticipée ($p_{futur}$) pour la régulation du débit volumétrique maximal ($Q_{max}$) dans le premier circuit de régulation (11) est utilisée uniquement dans des phases de pression déterminées et ignorée ou non utilisée dans d'autres phases de pression.

4. Dispositif de régulation (8) selon la revendication 3, dans lequel une première phase de pression (I) est **caractérisée par** une perte de pression rapide, une deuxième phase de pression (II) est **caractérisée par** une augmentation de pression rapide, une troisième phase de pression (III) est **caractérisée par** une augmentation de pression plate, une quatrième phase de pression (IV) est **caractérisée par** une augmentation de pression modérée (IV) et une cinquième phase de pression (V) est **caractérisée par** un plateau de pression avec une pression essentiellement constante, et chaque phase de pression est détectée par la détection de phase de pression (12).

5. Dispositif de régulation (8) selon la revendication 4, **caractérisé en ce que** dans le premier circuit de régulation (11) la pression anticipée ($p_{futur}$) pour la délimitation du débit volumétrique maximal ($Q_{max}$) est utilisée uniquement dans la quatrième phase de pression (IV), dans lequel la quatrième phase de pression (IV) se déplace de préférence et par approximation dans une plage du cycle de pression (P) de 50 % à 80 % d'un avancement de phase du cycle de pression (P).

6. Dispositif de régulation (8) selon l'une des revendications 2 à 5, **caractérisé en ce que**, dans le processus de régulation, la pression anticipée ($p_{futur}$) est déterminée, en tant qu'extrapolation de préférence linéaire de l'augmentation de pression ($p_{ist}$) en cours, à un avancement de phase ($\varphi_{87\%}$) déterminé par calcul du cycle de pression (P).

7. Dispositif de régulation (8) selon la revendication 6, **caractérisé en ce que**, dans le processus de régulation, l'avancement de phase ($\varphi_{87\%}$) déterminé par calcul auquel la pression anticipée ($p_{futur}$) est déterminée ne coïncide pas avec un avancement de phase ($\varphi_{81\%}$) effectif auquel survient la pression maximale ($p_{max}$) effective d'un cycle de pression (P).

8. Dispositif de régulation (8) selon l'une des revendications précédentes, **caractérisé en ce que** le premier circuit de régulation (11) comprend un filtre à valeur moyenne qui ajuste le débit volumétrique maximal ($Q_{max}$) en fonction d'une pression maximale ($p_{max}$) d'un cycle de pression (P) précédant directement le cycle de pression (P) en cours, laquelle pression maximale est mise en cache dans le dispositif de régulation (8).

9. Dispositif de régulation (8) selon l'une des revendications 3 à 8, **caractérisé en ce que** le premier circuit de régulation (11) est un régulateur PID avec un organe proportionnel (P), un organe intégral (I) et un organe différentiel (D), dans lequel l'organe différentiel est de préférence mis à zéro dans des phases de pression déterminées, en particulier dans la première phase de pression (I), la deuxième phase de pression (II), la troisième phase de pression (III) et la cinquième phase de pression (V).

10. Dispositif de régulation (8) selon l'une des revendications précédentes, **caractérisé en ce que** le dispositif de régulation (8) comprend un pilotage pour le lissage de pointes de pression de la pression ($p_{ist}$) dans chacun des cycles de pression (P), dans lequel ce pilotage est configuré en tant qu'adaptateur de vitesse via lequel respectivement à un avancement déterminé du cycle de pression une vitesse de consigne ($\omega_{soll}$) d'un élément d'acheminement (5-1, 5-2, 5-3) de la pompe péristaltique (5) par rapport à une vitesse de consigne moyenne est réduite, avant achèvement d'un cycle de pression (P), d'une quantité déterminée à une vitesse de consigne diminuée ($\omega$-) et/ou est accrue, après achèvement d'un cycle de pression (P), d'une quantité déterminée à une vitesse de consigne accrue ($\omega$+) et respectivement maintenue pendant une durée de maintien déterminée de sorte que la vitesse ($\omega$) d'un élément d'acheminement (5-1, 5-2, 5-3) après achèvement d'un cycle de pression (P) soit accrue par à-coups de la quantité définie et ensuite réduite de façon linéaire jusqu'à la vitesse de consigne diminuée ($\omega$-) de l'élément d'acheminement (5-1, 5-2, 5-3).

11. Dispositif de régulation (8) selon l'une des revendications précédentes, **caractérisé en ce que** le processus d'acheminement comprend plus d'un, en particulier plus de 5 et de façon particulièrement préférée plus de 10 cycles de pression.

12. Pompe péristaltique sous la forme d'une pompe à galets (5) avec un rotor (7) rotatif et un dispositif de régulation (8) selon l'une des revendications précédentes,

dans laquelle les éléments d'acheminement (5-1, 5-2, 5-3) pour l'écrasement d'un tuyau à écraser (4) sont conçus en tant que galets agencés contre un rotor (7), dans laquelle le tuyau à écraser (4) est monté le long d'un lit de tuyau (6) avec une zone de pénétration (6-1) et une zone d'évacuation (6-2) et, lorsque le rotor (7) tourne, les éléments d'acheminement (5-1, 5-2, 5-3) écrasent respectivement de façon étanche aux fluides l'une derrière l'autre une section du tuyau à écraser (4) lors de l'entrée des éléments d'acheminement (5-1, 5-2, 5-3) dans le lit de tuyau (6) à hauteur de la zone de pénétration (6-1) et jusqu'à la sortie des éléments d'acheminement (5-1, 5-2, 5-3) hors du lit de tuyau (6) à hauteur de la zone d'évacuation (6-2), et ce faisant acheminent le milieu se trouvant dans le tuyau à écraser (4) jusque dans la conduite de distribution (9) dans la direction de rotation et à l'encontre d'une pression dynamique dans la conduite de distribution (9),

dans laquelle un volume respectif, du tuyau à écraser (4), se trouvant avant la zone d'évacuation (6-2) est comprimé depuis un volume maximal vers un volume minimal jusqu'à ce qu'un élément d'acheminement (5-1, 5-2, 5-3) sorte du lit de tuyau (6) à hauteur de la zone d'évacuation (6-2) et ce faisant libère la section du tuyau à écraser (4) écrasée par cet élément d'acheminement (5-1, 5-2, 5-3) et ainsi le volume, du tuyau à écraser (4), se trouvant avant la zone d'évacuation (6-2) est augmenté depuis le volume minimal vers le volume maximal, dans laquelle respectivement une réduction de volume et une augmentation de volume ultérieure du volume se trouvant avant la zone d'évacuation (6-2) constituent ensemble un cycle de pression (P) complet,

et la pompe à galets (5) présente n éléments d'acheminement (5-1, 5-2, 5-3) répartis régulièrement et de préférence radialement de sorte que la pompe à galets (5) exécute n courses de pompe avec n cycles de pression (P) lors d'une rotation complète du rotor (7) de 360°,

et chaque phase de pression (I à V) d'un cycle de pression (P) correspond essentiellement à une plage angulaire déterminée d'une position angulaire ($\varphi$) d'un élément d'acheminement (5-1, 5-2, 5-3).

13. Pompe péristaltique selon la revendication 12, **caractérisée en ce que** le début d'au moins une phase de pression (I à V) d'un cycle de pression (P) est indirectement déterminé par la détection de phase de pression (12) via une position angulaire ($\varphi$) d'un élément d'acheminement (5-1, 5-2, 5-3).

14. Appareil d'injection (1) pour l'injection d'un moyen d'injection dans un corps animal ou humain au moyen d'une pompe péristaltique conçue en tant que pompe à galets (5), **caractérisé en ce que** l'appareil d'injection (1) contient une pompe péristaltique (5) selon l'une des revendications 12 ou 13.

15. Procédé de régulation pour une pompe péristaltique (5) avec un tuyau à écraser (4) et des éléments d'acheminement (5-1, 5-2, 5-3) se déplaçant de façon cyclique pour l'acheminement d'un milieu guidé dans le tuyau à écraser (4) pendant un processus d'acheminement avec un débit volumétrique commandé jusque dans une conduite de distribution (9) reliée au tuyau à écraser (4), dans lequel les éléments d'acheminement (5-1, 5-2, 5-3) compriment le tuyau à écraser (4) de façon cyclique de sorte qu'un tracé de pression d'une pression ($p_{ist}$) se règle dans la conduite de distribution (9), lequel présente des cycles de pression (P) se renouvelant de façon cyclique, dans lequel chaque cycle de pression présente un minimum de pression, une augmentation de pression, un maximum de pression et une baisse de pression, dans lequel le procédé de régulation régule une vitesse de la pompe péristaltique (5) de sorte qu'un débit volumétrique maximal ($Q_{max}$) soit atteint sans ce faisant dépasser une limite de pression ($p_{Grenz}$) dans la conduite de distribution (9), dans lequel le procédé de régulation présente un premier circuit de régulation (11) pour la régulation du débit volumétrique maximal ($Q_{max}$) qui obtient un débit volumétrique de consigne ($Q_{soll}$) et la limite de pression ($p_{Grenz}$) en tant que grandeurs de guidage, dans lequel le procédé est **caractérisé en ce que**

pour chaque cycle de pression (P) est calculée une pression anticipée ($p_{futur}$) pour une pression maximale ($p_{max}$) à escompter à l'intérieur du cycle de pression (P) sur la base de la pression ($p_{ist}$) dans la conduite de distribution (9), et le débit volumétrique maximal ($Q_{max}$) est délimité en tenant compte de la pression anticipée ($p_{futur}$) de sorte que la pression ($p_{ist}$) dans la conduite de distribution ne dépasse pas la limite de pression ($p_{Grenz}$) dans le cycle de pression (P).

2a,b,c

zu 9

3"

3'

3

5-1

5

4

1

8

**Fig. 1**

**Fig. 2**

$p_{Gefährdungsdruck}$

$p_{ist}$ ————

$p_{Grenz}$

$p_{max}$

$p'_{max}$

I

II

III

IV

V

P

$p_{min}$

P'

$p'_{min}$

P"

$p''_{min}$

EP 4 249 751 B1

$\varphi_{i°}=120°$

100%

$\varphi_{i°}$

Fig. 3

Fig. 4

Fig. 5

Fig. 6

Raise %

Flow

-Raise %

$Q_+$

$Q_-$

$Q_{Mittel}$ bzw. $Q_{Soll}$

I

II

III

IV

V

I'

P

x_hold

50%

1-x_hold

100%

Fig. 7A

Fig. 7B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9567992 B2
- US 6673033 B1

- DE 102013113387 A1